# EUROPEAN PATENT APPLICATION

(11) **EP 4 796 213 A1**
(43) Date of publication of application: **26.08.2026**
(21) Application number: 24880191.2
(22) Date of filing: 18.10.2024
(51) Int. Cl.: B01J 13/00, B01J 13/06, C08J 3/075, A61K 9/50, A61K 47/69, A61K 8/04, A61K 8/11, A61L 27/52

(54) **BIO-PARTICLE ENCAPSULATED HYDROGEL USING AQUEOUS-TWO PHASE SYSTEM AND METHOD FOR PRODUCING SAME**

(30) Priority: 19.10.2023 KR 20230140541
(71) Applicant: Pnaseer Inc., Suwon-si Gyeonggi-do 16514 (KR)
(72) Inventor: SHIN, Hyunwoo, Suwon-si Gyeonggi-do 16508 (KR)
(74) Representative: Müller, Christian Stefan Gerd
(86) International application number: PCT/KR2024/015895
(87) International publication number: WO 2025/084854

(57) **Abstract**

While conventional hydrogel manufacturing methods employing physical or chemical stimuli require sophisticated processes and can compromise the activity and structural integrity of particles-particularly when biological materials sensitive to external reactions are involved-the hydrogel and the preparation method thereof using an Aqueous Two-Phase System (ATPS) provided by the present invention enable the simultaneous formation of the hydrogel and encapsulation of desired nanoparticles within the hydrogel. The present invention successfully achieves the concentration of desired nanoparticles within a hydrogel with high yields through a simplified process. Furthermore, by facilitating concurrent concentration and hydrogel formation, the present invention relates to a hydrogel and a preparation method thereof using an ATPS that significantly reduces manufacturing time and costs.

## Description

### Technical Field

This present invention relates to innovative new drugs or next-generation biopharmaceuticals associated with the Fourth Industrial Revolution. Specifically, it concerns a bio-particle encapsulating hydrogel using an Aqueous Two-Phase System (ATPS), and its manufacturing method, which can be utilized as a delivery platform for various new drugs and for vaccine formulation.

### Background Art

An Aqueous Two-Phase System (ATPS) is a method for concentrating and extracting particles by creating two aqueous phases. It is formed when two or more polymers, or one polymer and one kosmotropic salt, or two salts (one chaotropic salt and one kosmotropic salt) form when mixed at appropriate concentrations or specific temperatures. An ATPS, composed mostly of water and non-volatile components, have been used for years in biotechnology applications as non-denaturing and benign separation media. However, the type and concentration of substances used to form the ATPS are critical for effectively concentrating and extracting target materials.

Furthermore, an ATPS can be applied not only to particle separation but also to hydrogel formation. Hydrogels are materials formed when the chains of hydrophilic polymers cross-link to create a three-dimensional network structure. They possess unique properties, such as containing large amounts of water internally and achieving thermodynamic stability in aqueous solutions, making them widely utilized in diverse fields including medicine, cosmetics, and industry. It has been reported that using an ATPS can create micro-sized hydrogel particles or induce a sol-gel state to produce injectable hydrogels.

However, the theoretical interpretation of hydrogel formation using an ATPS is complex, limiting its application. An ATPS allows for the formation of hydrogels while concentrating desired bio-particles within them. To achieve this, an understanding of the principles governing hydrogel formation via this method is essential. Based on this understanding, meticulous design of the types and concentrations of substances constituting the ATPS is required. Based on currently known interpretation methods, significant difficulties exist in simultaneously concentrating target particles using the Aqueous Two-Phase System and manufacturing hydrogels containing those target particles.

### DISCLOSURE

### Technical Problem

The present invention has been made to solve the aforementioned problems. An object of the present invention is to provide a method for preparing a hydrogel containing target particles while simultaneously concentrating the target particles using an Aqueous Two-Phase System (ATPS). Specifically, the object of the present invention is to provide a method for preparing a particle-concentrated hydrogel through hyaluronic acid encapsulation.

Another object of the present invention is to provide a hydrogel prepared according to the aforementioned method, as well as a method for skin aesthetic treatment, a composition for skin aesthetics, and a composition for wound healing, each comprising the hydrogel.

Still another object of the present invention is to provide a virus-encapsulated hydrogel for vaccines and an anti-cancer agent-encapsulated hydrogel, both of which are prepared according to the aforementioned method.

Still another object of the present invention is to provide a container for extracting or concentrating particles used in preparing a hydrogel according to the aforementioned method, the container comprising: a body providing an internal space configured to accommodate an aqueous solution for an ATPS and to extract or concentrate the particles therein during centrifugation; a first lid provided at one side of the body for injecting blood or a separate substance into the internal space of the body; and a second lid provided at the other side of the body opposite to the one side for discharging the particles extracted or concentrated in the body.

However, the technical problems to be addressed by the present invention are not limited to the problems mentioned above, and other technical problems not mentioned will be clearly understood by those of ordinary skill in the art from the following description.

### Technical Solution

Hereinafter, various embodiments described herein will be described with reference to figures. In the following description, numerous specific details are set forth, such as specific configurations, compositions, and processes, etc., in order to provide a thorough understanding of the present invention. However, certain embodiments may be practiced without one or more of these specific details, or in combination with other known methods and configurations. In other instances, known processes and preparation techniques have not been described in particular detail in order to not unnecessarily obscure the present invention. Reference throughout this specification to "one embodiment" or "an embodiment" means that a particular feature, configuration, composition, or characteristic described in connection with the embodiment is included in at least one embodiment of the present invention. Thus, the appearances of the phrase "in one embodiment" or "an embodiment" in various places throughout this specification are not necessarily referring to the same embodiment of the present invention. Additionally, the particular features, configurations, compositions, or characteristics may be combined in any suitable manner in one or more embodiments.

Generally, to manufacture a hydrogel, physical stimuli or chemical stimuli are applied to a material serving as the hydrogel's backbone (e.g., hyaluronic acid, collagen, chitosan, etc.), causing cross-linking to occur between the materials serving as the backbone. However, when bio-particles or substances sensitive to external reactions are incorporated into the hydrogel, conventional physical or chemical stimuli may affect the particles within the hydrogel. Existing commercial products primarily manufacture hydrogels by adding chemicals that induce cross-linking. When used for medical purposes, such as therapeutic agents, safety concerns related to these chemicals may arise.

Furthermore, to apply hydrogels to various therapeutic fields, it is essential to prepare injectable hydrogels that can be administered into the human body via injection. However, cross-linking through physical or chemical stimuli requires sophisticated processes to manufacture such injectable hydrogels, which poses a significant limitation to their practical application.

Numerous studies have been conducted to date because using an ATPS allows for simple and rapid hydrogel formation without adversely affecting desired target particles. However, due to the complexity of theoretical interpretations, conventional research has focused primarily on hydrogel formation itself using an ATPS. Consequently, the approach of applying the particle separation characteristics of an ATPS to hydrogel formation-thereby simultaneously achieving the encapsulation of target particles and hydrogel formation-has remained largely unexplored.

In the present invention, by configuring one aqueous phase of the ATPS to consist of a material serving as a backbone (or scaffold) of the hydrogel, the backbone material is present at a locally high concentration. In this state, the backbone material is densely packed due to the high concentration, thereby facilitating cross-linking.

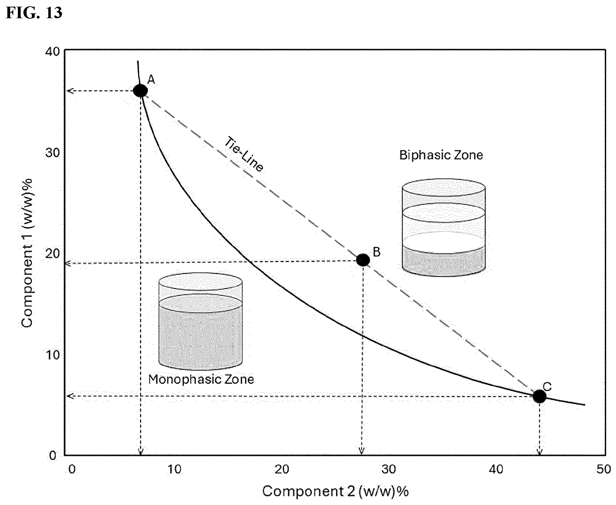

For example, as shown in FIG. 13, when an aqueous solution having composition B (12%, 22%) is prepared, it is partitioned into aqueous solutions having composition A (28%, 8%) and composition C (4%, 38%) upon phase separation, during which the concentration of the material constituting the aqueous phase locally increases.

Upon formation of the ATPS, the backbone material, which previously existed in a sol state, undergoes a sol-gel process as its local concentration increases. The aqueous phase during the sol-gel process can be easily converted into a gel-state hydrogel even through a slight physical stimulus. By utilizing these characteristics, an injectable aqueous phase in a sol-gel process can be prepared using the ATPS and subsequently injected into the body, where a gel-state hydrogel is formed by physiological temperature (body heat).

By applying the aforementioned analytical method, it is possible to simultaneously perform the encapsulation of nanoparticles within a hydrogel and hydrogel formation. For example, when an ATPS is formed using hyaluronic acid and polyethylene glycol (PEG), the hyaluronic acid aqueous phase, which initially existed in a sol state, undergoes a sol-gel process. If biological particle A, which has a strong affinity for the hyaluronic acid aqueous phase, is present, particle A undergoes the sol-gel process together while being concentrated within said hyaluronic acid aqueous phase. Therefore, by utilizing the affinity between biological particle A and the material serving as a backbone (or scaffold), it is possible to encapsulate particle A and form a hydrogel simultaneously using an ATPS.

### 1. Method for Preparing a Hydrogel Using an Aqueous Two-Phase System (ATPS)

In one embodiment of the present invention, there is provided a method for preparing a hydrogel, the method comprising: 1) preparing a first aqueous solution comprising a biological material and a second aqueous solution; 2) mixing the first aqueous solution and the second aqueous solution to form an ATPS; and 3) forming a particle-concentrated hydrogel through encapsulation.

In one embodiment of the present invention, there is provided a method for preparing a hydrogel, the method comprising: 1) mixing a first aqueous solution and a second aqueous solution in the presence of a cationic salt to form an ATPS; and 2) forming a porous hydrogel structure in one of the phases through the ATPS.

In another embodiment of the present invention, there is provided a method for preparing a hydrogel, further comprising: 3) forming a hydrogel in which a biological material is incorporated into the porous hydrogel structure.

As used herein, the term "Aqueous Two-Phase System (ATPS)" refers to a system formed of two aqueous immiscible solutions capable of liquid-liquid partitioning, which can effectively extract, separate, purify, and concentrate proteins, membranes, viruses, enzymes, nucleic acids, and other biomolecules. However, due to the nonspecific separation mechanism employed by the aqueous two-phase system process, there have been cases where only modest purification factors were obtained.

Primary processing parameters related to such an ATPS are all associated with influencing the partitioning behavior of components within a process stream. For example, whether a molecule partitions into a top or bottom phase is determined by molecular characteristics (e.g., charge, molecular weight, and solvation) and polymer characteristics (e.g., concentration, molecular weight, and hydrophobicity). Furthermore, the physicochemical environment of the system (e.g., temperature, pH, and ionic strength) also affects the yield and purification factors of the concentration and extraction processes conducted by the ATPS. Ensuring optimal performance requires a careful balance and consideration of various interaction factors among different operating parameters. Additionally, due to a lack of fundamental knowledge regarding the partitioning of biological components in an ATPS, it is often difficult to obtain only the desired target material with high yields. Consequently, process optimization necessitates the adoption of an experimental design approach based on fundamental knowledge of biological components, along with extensive screening of various conditions. As a result, the comprehensive development of a new ATPS process is a time-consuming and labor-intensive task.

Despite these limitations, the ATPS according to the present invention creates two aqueous phases to concentrate and extract particles. It can simultaneously concentrate the target particles and manufacture a hydrogel containing these particles at a high yield.

In the present invention, notwithstanding the need for a careful balance and consideration of various interaction factors among different operating parameters to form an ATPS, it has been confirmed that a binodal curve-which enables the formation of a hydrogel with encapsulated biological materials-can be established based on the ratio of the average molecular weight (kDa) of the solute in the first aqueous solution to the weight percentage (w/w) of the solute in the second aqueous solution. Through the binodal curve, a hydrogel having biological materials encapsulated therein can be formed by controlling the ratio of the average molecular weight (kDa) of the solute in the first aqueous solution to the weight percentage (w/w) of the solute in the second aqueous solution. Furthermore, those of ordinary skill in the art will understand that a hydrogel with encapsulated biological materials can be formed in view of the weight percentage of cations, the type of cations, the average molecular weight (kDa) of the solute in the first aqueous solution, and the weight percentage (w/w) of the solute in the second aqueous solution. For example, it will be understood that the hydrogel can be formed by considering the ratio of the weight percentage of cations to the average molecular weight (kDa) of the solute in the first aqueous solution, or the ratio of the weight percentage of cations to the weight percentage (w/w) of the solute in the second aqueous solution.

As used herein, the term "hydrogel" refers to a material that maintains a three-dimensional structure by swelling upon absorbing a large amount of water or body fluid into a cross-linked lattice within the water or body fluid. Even after being swollen, the hydrogel remains thermodynamically stable and possesses mechanical and physicochemical properties corresponding to an intermediate state between a liquid and a solid. Such hydrogels generally exhibit biocompatibility, high porosity, and oxygen permeability, and can demonstrate physical properties similar to those of biological soft tissues.

In another embodiment of the present invention, there is provided the method for preparing a hydrogel, wherein the solute of the first aqueous solution is selected from: A natural polymer group consisting of hyaluronic acid, gelatin, alginate, xanthan gum, chitosan, pectin, galactomannan, glucomannan, fibrin, collagen, dextran (DEX), carboxymethyl cellulose (CMC), agar, psyllium husk, methylcellulose, poly(lysine), and dextran succinic acid; and a synthetic polymer group consisting of polyacrylamide (PAM), carbomer, polyacrylic acid (PAA), poly(lactide-co-glycolide) (PLGA), poly(2-hydroxyethyl methacrylate) (PHEMA), polylactic acid (PLA), polyurethane, poly(N,N-dimethylacrylamide) (PDMAAm), poly(glycerol sebacate) (PGS), poly(glycolyglucan), poly(acryloyl-glucosamine), poly(acryloyloxyethyltrimethyl ammonium chloride) (PAE), furan derivatives, and poly(N,N-bis(methacryloyl)amino acid).

As used herein, the term "hyaluronic acid" (also referred to as "HA") is a linear polysaccharide consisting of alternating glycosidic bonds between β-D-glucuronic acid and β-D-N-acetylglucosamine. HA is a biodegradable and biocompatible natural polymer that exists widely in nature and has a wide molecular weight distribution ranging from 1,000 Da to 50,000,000 Da. In one embodiment of the present invention, the average molecular weight of the hyaluronic acid may range from a minimum of 1 kDa to a maximum of 50,000 kDa. Specifically, the average molecular weight may be 10-50,000 kDa, 20-50,000 kDa, 40-50,000 kDa, 100-50,000 kDa, 1000-50,000 kDa, 2000-50,000 kDa, 10-10,000 kDa, 20-10,000 kDa, 40-10,000 kDa, 100-10,000 kDa, 1000-10,000 kDa, 2000-10,000 kDa, 10-5,000 kDa, 20-5,000 kDa, 40-5,000 kDa, 100-5,000 kDa, 1000-5,000 kDa, or 2000-5,000 kDa, without being limited thereto.

This hyaluronic acid forms a highly viscous gel by binding large amounts of water. It is abundantly present in the vitreous humor of the eye, synovial fluid, cartilage, and skin. It is known to play a protective role by lubricating joints, imparting skin elasticity, and preventing bacterial penetration into the skin due to its high viscosity. Therefore, it has been used in numerous fields to date, including degenerative arthritis, cataracts, wrinkle improvement, drug delivery, stem cell scaffolds, and cosmetic moisturization. Furthermore, it is abundantly present in the extracellular matrix of tissues, regulating cell differentiation and proliferation, and exhibiting wound healing effects (Erika Nyman et al., J. Plast Surg Hand Surg. 2013; 47; 89-92) and wound healing mechanisms (Richard D Price et al., Am J Clin Dermatol; 2005; 6(6) 393-402) have been reported.

Recent studies have also reported the pharmacological action of low-molecular-weight hyaluronic acid in inducing vascular regeneration around damaged tissue, thereby enabling efficient wound healing. Based on these properties, hyaluronic acid is widely utilized in wound treatments, arthritis therapies, drug delivery systems, and tissue engineering. Furthermore, due to its excellent biocompatibility, biodegradability, and ability to prevent tissue adhesion, it is also used as an anti-adhesion agent in its own right.

As used herein, the term "collagen" refers to a scleroprotein primarily present in animal bones and skin, also distributed in cartilage, organ membranes, hair, and the like, and is a component of fish scales. It is also referred to as a collagenous substance and exists as a fibrous solid. Under an electron microscope, collagen exhibits a complex transverse banding structure. While insoluble in water, dilute acids, and dilute alkalis, it dissolves into gelatin upon boiling. Structurally, collagen is a triple helix composed of three intertwined polypeptide chains and is characterized by a particularly high content of hydroxyproline. The amino acid sequence of collagen typically follows patterns such as 'Glycine-Proline-X' or 'Glycine-Amino Acid-Hydroxyproline,' with glycine, hydroxyproline, and proline being its primary constituent amino acids. Furthermore, collagen is resistant to proteolytic enzymes such as trypsin but is susceptible to the action of collagenase. A method for isolating collagen from tissues involves extraction with organic solvents, followed by acid/alkali treatment and subsequent treatment with trypsin and hyaluronidase, thereby obtaining collagen as an insoluble substance. Collagen is a representative biodegradable polymer and has been widely used as a scaffold in tissue engineering. Although insoluble, collagen offers advantages such as excellent stability as a biological component and low immunogenicity due to its high homology across different animal species.

As used herein, the term "chitosan" refers to an excellent natural raw material for hydrogels due to its biocompatibility, biodegradability, and cost-effectiveness. Chitosan is a linear polysaccharide composed of β-(1→4)-linked D-glucosamine (deacetylated unit) and N-acetyl-D-glucosamine (acetylated unit). It is produced by treating chitin shells from crustaceans, such as shrimp, with an alkaline substance such as sodium hydroxide (*NaOH*). Chitosan is useful for medical applications, such as hemostatic bandages to reduce bleeding, and is also effective as an antibacterial agent. Furthermore, it has been utilized in the field of transdermal drug delivery. The sol-gel transition of a chitosan hydrogel occurs very readily under mild conditions in the presence of divalent cations acting as ionic cross-linkers. However, it has been observed that chitosan hydrogels cross-linked by ionic bonds lose mechanical strength over time due to the natural release of ions from the matrix. On the other hand, a hydrogel structure cross-linked by covalent bonds, which can maintain its mechanical properties over a long period, is prepared by modifying the hydroxyl and amine groups of the chitosan.

As used herein, the term "dextran" refers to a polymer in which glucose units are linked by α-1,6 glycosidic bonds, characterized by excellent water solubility and biocompatibility. Due to these properties, dextran possesses the ability to effectively absorb and retain moisture, making it widely utilized as a fundamental component of hydrogels. To form a hydrogel, dextran typically establishes bonds between polymer chains using a cross-linking agent. During this process, a three-dimensional network structure is generated through a reaction between the hydroxyl groups of the dextran and the cross-linking agent. This network structure creates internal spaces capable of absorbing and maintaining water, thereby imparting elastic and flexible characteristics to the hydrogel. Dextran-based hydrogels are employed in various applications, including drug delivery systems, wound dressings, and tissue engineering. In drug delivery systems, they provide the functional capability to effectively encapsulate drugs and release them when required. Furthermore, due to the inherent biocompatibility of dextran, such hydrogels exhibit minimal side effects in the human body, ensuring safe clinical use.

As used herein, the term "alginate" refers to a natural polysaccharide extracted from seaweed and is a material highly suitable for hydrogel formation. Alginate primarily consists of a block structure of β-D-mannuronic acid and α-L-guluronic acid, and possesses an excellent ability to absorb moisture and form a gel state. To form a hydrogel using alginate, an ion exchange reaction is generally employed. In this process, the alginate reacts with ions such as calcium ions (*Ca*²⁺) to create a cross-linked structure, thereby forming a three-dimensional network. This network structure is formed by bonds between the hydroxyl groups of the alginate and the calcium ions, which imparts the ability to absorb and maintain moisture to the hydrogel. Alginate-based hydrogels are widely utilized in drug delivery systems, wound dressings, and tissue engineering. Particularly in drug delivery systems, they can effectively encapsulate drugs and control release rates according to environmental changes, thereby contributing to increased therapeutic efficiency. Furthermore, alginate exhibits excellent biocompatibility and can be safely used in the human body.

As used herein, the term "carboxymethyl cellulose" (also referred to as "CMC") is a derivative of cellulose characterized by excellent water solubility and high viscosity. CMC is typically produced by chemically modifying cellulose extracted from plants and is a material highly suitable for hydrogel formation. Because CMC contains hydroxyl groups and carboxyl groups (-*COOH*), it exhibits superior interaction with water, providing an excellent ability to absorb and retain moisture. To form a hydrogel with CMC, a cross-linking agent is generally used to promote bonding between CMC molecules. In this process, a three-dimensional network structure is generated through a reaction between the carboxyl groups of the CMC and the cross-linking agent. This network serves to entrap water and impart specific physical properties to the hydrogel. CMC hydrogels swell upon absorbing water and, due to these characteristics, maintain a flexible and elastic structure.

As used herein, the term "polyacrylamide" (also referred to as "PAM") is a polymer of acrylamide characterized by high water absorption capacity and flexibility. PAM is generally water-soluble and is widely utilized to form hydrogels in various applications. Due to the inherent properties of PAM, these hydrogels possess excellent physical characteristics, making them highly useful for biological and industrial applications. To form a hydrogel with PAM, a cross-linking agent is typically employed to promote bonding between polymer chains. In this process, the amide groups of the acrylamide react with the cross-linking agent to create a three-dimensional network structure. This network structure imparts the ability to absorb and retain water to the PAM, while providing flexibility and elasticity to the resulting hydrogel. PAM-based hydrogels are utilized in various fields, such as drug delivery systems, wound dressings, and environmental monitoring. Particularly in drug delivery systems, they contribute to enhancing therapeutic efficiency by providing the functional capability to effectively encapsulate drugs and release them as needed. Furthermore, PAM exhibits excellent biocompatibility, allowing it to be safely used in the human body.

As used herein, the term "poly(vinyl alcohol)" (also referred to as "PVA") is a polymer of vinyl alcohol and is a water-soluble polymeric material characterized by excellent mechanical properties and water resistance. PVA exhibits superior biocompatibility and allows for various chemical modifications, making it widely utilized as a suitable material for hydrogel formation. To form a hydrogel using PVA, a cross-linking agent is typically employed. In this process, the hydroxyl groups (-OH) of the PVA react with the cross-linking agent to generate a three-dimensional network structure. This network provides internal spaces capable of absorbing and retaining water, thereby imparting flexibility and elasticity to the resulting hydrogel. PVA-based hydrogels swell upon effectively absorbing moisture, which allows them to maintain stable physical properties across diverse environments. These PVA-based hydrogels are utilized in various fields, including: Drug delivery systems, Wound dressings, Tissue engineering, and Cosmetics. Particularly in drug delivery systems, they contribute to enhancing therapeutic efficiency by providing the functional capability to effectively encapsulate drugs and release them as needed.

The solute of the first aqueous solution for forming the ATPS is not particularly limited as long as it is capable of forming a hydrogel, regardless of whether it is a natural polymer or a synthetic polymer. Those of ordinary skill in the art will understand that materials capable of forming a porous hydrogel structure-such as hyaluronic acid, collagen, chitosan, dextran (DEX), alginate, carboxymethyl cellulose (CMC), polyacrylamide (PAM), poly(vinyl alcohol) (PVA), and the like-may be included therein.

In another embodiment of the present invention, there is provided a method for preparing a hydrogel, wherein the solute of the second aqueous solution is at least one selected from the group consisting of: polyethylene glycol (PEG), propylene glycol (PPG), polyvinylpyrrolidone (PVP), poly(vinyl alcohol) (PVOH, PVA, or PVAI), poly(vinyl methyl ether) (PVME), hydroxypropyl starch (HP-starch), UCON, polyethersulfone (PES), pullulan, polypropylene glycol dimethyl ether (PPGDME), dextran (DEX), maltodextrin, hydroxyethyl cellulose (HEC; e.g., Natrosol), polyethylene glycol methyl ether (PEGME), polyethylene glycol dimethyl ether (PEGDME), polyacrylamide (PAM), poly(styrene sulfonate) (PSS), DEAE-dextran, polyethyleneimine (PEI), hydroxypropyl methylcellulose (HPMC), poly(N-isopropylacrylamide) (PNIPAM), poly(diallyldimethylammonium chloride) (PDADMAC), poly(vinylcaprolactam) (PVCL), and Ficoll.

The solute of the second aqueous solution is not particularly limited as long as it is capable of forming an ATPS with the first aqueous solution and is capable of forming a hydrogel. Those of ordinary skill in the art will understand that the material is not particularly limited as long as it is capable of forming a porous hydrogel structure.

In one embodiment of the present invention, there is provided a method for preparing a hydrogel, wherein the salt is a salt of a cation selected from the group consisting of *Na*⁺, *K*⁺, *Ca*²⁺, *Mg*²⁺, *Ba*²⁺, *Ag*⁺, *Pb*²⁺, and *Al*³⁺ and other anions, and the salt is present in an amount of about 0.5% to about 20% (w/w). In one embodiment, the weight percentage of the salt corresponds to a range from a minimum of about 0.5% (w/w) to a maximum of about 20% (w/w), and may specifically be 0.5-20% (w/w), 1-20% (w/w), 2-20% (w/w), 3-20% (w/w), 4-20% (w/w), 5-20% (w/w), 0.5-15% (w/w), 1-15% (w/w), 2-15% (w/w), 3-15% (w/w), 4-15% (w/w), or 5-15% (w/w), without being limited thereto.

In one embodiment of the present invention, there is provided a method for preparing a hydrogel, wherein the weight percentage of the polyethylene glycol (PEG) is about 3% to about 50% (w/w). In one embodiment, the weight percentage of the PEG ranges from a minimum of about 3% (w/w) to a maximum of about 50% (w/w), and may specifically be 3-50% (w/w), 4-50% (w/w), 5-50% (w/w), 6-50% (w/w), 7-50% (w/w), 8-50% (w/w), 3-45% (w/w), 4-45% (w/w), 5-45% (w/w), 6-45% (w/w), 7-45% (w/w), 8-45% (w/w), 3-20% (w/w), 4-20% (w/w), 5-20% (w/w), 6-20% (w/w), 7-20% (w/w), 8-20% (w/w), 3-15% (w/w), 4-15% (w/w), 5-15% (w/w), 6-15% (w/w), 7-15% (w/w), or 8-15% (w/w), without being limited thereto.

In one embodiment of the present invention, there is provided a method for preparing a hydrogel, wherein the weight percentage of the hyaluronic acid (HA) is about 0.1% to about 5% (w/w). In one embodiment, the weight percentage of the hyaluronic acid may be 0.1-4% (w/w), 0.1-3% (w/w), 0.5-5% (w/w), 0.5-4% (w/w), 0.5-3% (w/w), 1-5% (w/w), or 2-5% (w/w), without being limited thereto.

In one embodiment of the present invention, there is provided a method for preparing a hydrogel, wherein the concentration of the hyaluronic acid (HA) is about 0.10% by weight (wt%) or more. In another embodiment of the present invention, there is provided a method for preparing a hydrogel, wherein the concentration of the hyaluronic acid (HA) is about 0.15% by weight (wt%) or more. In still another embodiment of the present invention, there is provided a method for preparing a hydrogel, wherein the concentration of the hyaluronic acid (HA) is about 0.16% by weight (wt%) or more.

In one embodiment of the present invention, there is provided a method for preparing a hydrogel, wherein the first aqueous solution comprises at least one selected from the group consisting of: mesenchymal stem cells (MSCs), MSC-derived exosomes, viruses, proteins, RNA, platelet-rich plasma (PRP), and regeneration factors.

As used herein, the term "platelet-rich plasma" (also referred to as "PRP") refers to a concentrate of platelets in plasma. Because PRP is highly enriched with growth factors capable of stimulating the migration and proliferation of bone-forming cells, it is frequently utilized in various clinical applications such as dental surgery and fracture healing. In particular, platelet-derived growth factor (PDGF) is present at high concentrations within PRP. Prior studies have demonstrated that PDGF provides chemotactic stimulation to SaOS-2 osteoblasts, inducing migration as observed in microchemotaxis assays. The hydrogel formed via the ATPS according to the present invention is capable of concentrating and incorporating PRP and growth factors, including PDGF, into the hydrogel matrix.

The hydrogel using the ATPS according to the present invention enables simple concentration of stem cells within the hydrogel without a crosslinker. Furthermore, the hydrogel using the ATPS according to the present invention enables simple concentration of viruses or exosomes present at low concentrations within the hydrogel without a crosslinker. The hydrogel using the ATPS according to the present invention can simply concentrate protein-based anticancer agents within the hydrogel without a crosslinker. Furthermore, the hydrogel using the ATPS according to the present invention can simply concentrate anticancer agents within the hydrogel without a crosslinker when applied to genome-based anticancer agents (such as siRNA).

Furthermore, when applying the hydrogel using the ATPS according to the present invention to vaccines, it is possible to simply concentrate the vaccine within the hydrogel without a crosslinking agent. For vaccines, side effects due to high concentrations are a major concern. Encapsulating the vaccine within the hydrogel allows for its slow release from the hydrogel, enabling a longer-lasting vaccine effect and offering the advantage of reduced side effects due to increased human body reactivity.

As used herein, the antigen used in the vaccine may be derived from a cell, a bacterium, a virus particle, or a part thereof. As defined herein, the antigen may be a protein, a peptide, a polysaccharide, a glycoprotein, a lipid, a nucleic acid, or a combination thereof that induces an immunogenic response in an animal, such as a mammal, a bird, or a fish. As defined herein, the immunogenic response may be humoral or cell-mediated. In cases where a substance that induces an immunogenic response is poorly antigenic, it may be conjugated to a carrier (such as albumin) or to a hapten using standard covalent bonding techniques-for example, by utilizing various commercially available reagent kits.

Furthermore, the vaccine composition of the present invention may further comprise an adjuvant. For example, the adjuvant may include at least one selected from the group consisting of a polysorbate-based non-ionic surfactant, aluminum hydroxide gel, squalene, and chitosan. In addition, the vaccine composition of the present invention may further comprise an adjuvant or an immune enhancer, and preferably, the immune enhancer may include at least one selected from the group consisting of a polysorbate-based non-ionic surfactant, aluminum hydroxide gel (e.g., *Al*(*OH*)₃ gel), squalene, and chitosan. Examples of the polysorbate-based non-ionic surfactant may include Tween 80 (polysorbate 80), Tween 60 (polysorbate 60), or Tween 20 (polysorbate 20), and more preferably, Tween 80. Additionally, the vaccine composition according to the present invention may further include additives such as an excipient and/or a stabilizer.

In one embodiment of the present invention, there is provided a method for preparing a hydrogel, wherein the regeneration factor is at least one selected from the group consisting of: bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

The regeneration factors or growth factors include various types, including but not limited to: BMP, FGF, VEGF, NGF, EGF, IGF, TGF, BDNF, PDGF, PlGF, and HGF. Among these regeneration factors, notable examples include EGF and PDGF. In particular, it is significant that the RRP is highly enriched with PDGF.

More specifically, the healing process of a wound or injury occurs through epithelialization, wound contraction, collagen synthesis, and the formation of granulation tissue. In full-thickness skin defect wounds, wound contraction plays a primary role in the healing process. In contrast, in partial-thickness defect wounds where skin appendages remain, healing through epithelialization is more significant than contraction. During wound healing, wound-healing cells migrate to the wound site, where they undergo division and proliferation triggered by several factors. These factors are known to include chemoattractants (e.g., attachment factors) and growth-promoting factors, which play integral roles in the healing process.

Among the various growth factors identified to date, EGF and PDGF are considered the most effective for wound healing and are highly suitable for clinical applications. Since its initial discovery in 1961, EGF has been demonstrated through both in vitro and in vivo studies to act as a potent mitogen for epithelial cells, thereby exerting a significant influence on epithelialization. PDGF, discovered in 1985, is known to induce chemotactic activity and mitogenic activity in fibroblasts, which in turn promotes collagen deposition and the formation of granulation tissue at the wound site. It is further understood that PDGF has a more profound effect on the proliferation and contraction phases of wound healing, rather than the epithelialization phase.

As used herein, the term "epidermal growth factor" (also referred to as "EGF") refers to a protein that stimulates cellular growth and differentiation by binding to its receptor, EGFR. Human EGF is a 6-kDa protein consisting of 53 amino acid residues and containing three intramolecular disulfide bonds. Among various regeneration factors, EGF is known to play a critical role in skin regeneration and is thus applied as an ingredient in functional cosmetics. Furthermore, EGF is not only listed in the International Cosmetic Ingredient Dictionary (ICID) of the Cosmetic, Toiletry, and Fragrance Association (CTFA) but has also been approved as a cosmetic ingredient by the Ministry of Food and Drug Safety (MFDS) in Korea, allowing for its official use as a cosmetic raw material both domestically and internationally (MFDS Notification No. 2006-12, April 12, 2006).

As used herein, the term "platelet-derived growth factor" (also referred to as "PDGF") is one of numerous growth factors that regulate cellular growth and division. In particular, PDGF plays a critical role in angiogenesis (including blood vessel growth from pre-existing vascular tissue) and the mitogenesis (i.e., proliferation) of mesenchymal cells, such as fibroblasts, osteoblasts, tenocytes, vascular smooth muscle cells, and mesenchymal stem cells (MSCs). Consequently, PDGF is utilized in medical fields for the treatment of chronic ulcers and in orthopedic and periodontal treatments as an alternative to bone autografts to stimulate bone regeneration and repair. Furthermore, PDGF is an essential protein for the cellular division of fibroblasts, a connective tissue cell type, during the wound healing process. Specifically, PDGF administered to monocyte-macrophages and fibroblasts stimulates chemotaxis, proliferation, and gene expression, while significantly increasing the influx of inflammatory cells and fibroblasts. This process accelerates the formation of extracellular matrix (ECM) and collagen, thereby shortening the overall time required for the healing process.

### 2. Cosmetic Method, Cosmetic, and Cosmetic Composition Comprising Hydrogel Prepared via ATPS

In one embodiment of the present invention, there is provided a skin cosmetic method, a skin cosmetic composition comprising a hydrogel prepared according to the aforementioned method, and a cosmetic product comprising said skin cosmetic composition.

The present invention provides a method for cosmetic applications using a hydrogel prepared via an ATPS, which further incorporates concentrated regeneration factor proteins. Specifically, the hydrogel may contain at least one concentrated regeneration factor selected from the group consisting of: BMP, FGF, VEGF, NGF, EGF, IGF, TGF, BDNF, PDGF, PlGF, and HGF.

In a preferred embodiment of the present invention, the skin cosmetic composition or cosmetic formulation is preferably an anti-aging cosmetic for the skin, but is not limited thereto.

In the present invention, the cosmetic composition may be manufactured in the form of: skin toner, nourishing lotion, nourishing essence, massage cream, cosmetic bath additives, body lotion, body milk, bath oil, baby oil, baby powder, shower gel, shower cream, sunscreen lotion, sunscreen cream, suntan cream, skin lotion, skin cream, UV-protecting cosmetics, cleansing milk, depilatory (cosmetic), face and body lotion, face and body cream, skin whitening cream, hand lotion, hair lotion, cosmetic cream, jasmine oil, bath soap, liquid soap, beauty soap, shampoo, hand cleanser (hand cleaner), medicated soap (non-medical), cream soap, facial wash, body wash, scalp cleanser, hair rinse, toilet soap, tooth whitening gel, and toothpaste. To this end, the composition of the present invention may further include solvents, appropriate carriers, excipients, or diluents commonly used in the manufacture of cosmetic composition.

The types of solvents that may be further included in the cosmetic composition of the present invention are not particularly limited, and for example, water, saline, dimethyl sulfoxide (DMSO), or combinations thereof may be used. The carriers, excipients, or diluents may include, but are not limited to, purified water, oils, waxes, fatty acids, fatty alcohols, fatty acid esters, surfactants, humectants, thickeners, antioxidants, viscosity stabilizers, chelating agents, buffering agents, and lower alcohols. In addition, the composition may further comprise, as necessary, a whitening agent, a moisturizing agent, a vitamin, a sunscreen agent, a perfume, a dye, an antibiotic, an antibacterial agent, and an antifungal agent.

Examples of the oil include hydrogenated vegetable oil, castor oil, cottonseed oil, olive oil, palm kernel oil, jojoba oil, and avocado oil. Examples of the wax include beeswax, spermaceti, carnauba wax, candelilla wax, montan wax, ceresin wax, liquid paraffin, and lanolin.

As the fatty acid, stearic acid, linoleic acid, linolenic acid, or oleic acid may be used. As the fatty alcohol, cetyl alcohol, octyldodecanol, oleyl alcohol, panthenol, lanolin alcohol, stearyl alcohol, or hexadecanol may be used. Examples of the fatty acid ester include isopropyl myristate, isopropyl palmitate, and butyl stearate.

As the surfactant, cationic surfactants, anionic surfactants, and non-ionic surfactants known in the art may be used, and surfactants derived from natural sources are preferred.

In addition, humectants, thickeners, antioxidants, and the like, which are widely known in the cosmetic field, may be included, and the types and amounts thereof are in accordance with those known in the art.

### 3. Composition for Wound Treatment and Regeneration Comprising Hydrogel Prepared via ATPS

In one embodiment of the present invention, there is provided a composition for wound treatment comprising a hydrogel prepared according to the aforementioned method.

As used herein, the term "wound" is preferably selected from the group consisting of: wounds (general trauma), pressure sores (decubitus ulcers), burns, abrasions, puncture ulcerative wounds, stab wounds, chronic skin wounds caused by reactive oxygen species (ROS), contusions, incisions, wounds of the throat or oral mucosa, lacerations, diabetic ulcers, leg ulcers, hypertensive ischemic ulcers, venous ulcers, and foot ulcers, but is not limited thereto.

Furthermore, the pharmaceutical composition of the present invention may further comprise appropriate carriers, excipients, or diluents according to conventional methods. Examples of the carriers, excipients, and diluents that may be included in the pharmaceutical composition of the present invention include: lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, methyl hydroxybenzoate, propyl hydroxybenzoate, talc, magnesium stearate, and mineral oil, but are not limited thereto.

In addition, the pharmaceutical composition according to the present invention may be formulated and used in the form of oral dosage forms (such as powders, granules, tablets, capsules, suspensions, emulsions, syrups, and aerosols), external preparations, suppositories, or sterile injectable solutions, each according to conventional methods known in the art.

More specifically, when formulating the composition, it may be prepared using diluents or excipients such as commonly used fillers, extenders, binders, wetting agents, disintegrants, and surfactants.

Solid dosage forms for oral administration include tablets, pills, powders, granules, and capsules. Such solid dosage forms may be prepared by mixing the pharmaceutical composition of the present invention with at least one excipient, such as starch, calcium carbonate (*CaCO*₃), sucrose, lactose, or gelatin. In addition to simple excipients, lubricants such as magnesium stearate and talc may also be utilized.

Liquid dosage forms for oral administration include suspensions, oral solutions, emulsions, and syrups. In addition to commonly used simple diluents such as water and liquid paraffin, various other excipients-for example, wetting agents, sweeteners, fragrances, and preservatives-may be included.

Preparations for parenteral administration include sterile aqueous solutions, non-aqueous solvents, suspensions, emulsions, lyophilized (freeze-dried) formulations, and suppositories.

As the non-aqueous solvents and suspending agents, propylene glycol, polyethylene glycol, vegetable oils such as olive oil, and injectable esters such as ethyl oleate may be used. Examples of the suppository bases include Witepsol, Macrogol, Tween 61, cocoa butter (theobroma oil), laurin fat (hard fat), and glycerinated gelatin.

The route of administration of the pharmaceutical composition according to the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, frequency of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

As used herein, the term "regeneration" refers to a condition or process in which tissue or organ repair and/or functional recovery is required. Specifically, the condition requiring regeneration is preferably selected from the group consisting of: Temporomandibular disorders (TMD); gastric ulcers caused by endoscopic submucosal dissection (ESD); human autologous fat grafting; the treatment of knee osteoarthritis (OA); the treatment of chronic non-healing ulcers; hair regeneration therapy for androgenetic alopecia; the treatment of Achilles tendon rupture; high-risk proliferative diabetic retinopathy; patellar dislocation; lumbar facet joint syndrome; retinitis pigmentosa; diabetic macular edema; traumatic bone cysts; scarring; dry eye syndrome; Sjogren's syndrome; osteoarthritis; meniscal lesions; melasma; muscle injury; ulnar neuropathy at the elbow; chronic ulcers; alopecia; epicondylitis; bone resorption; rotator cuff tears; thin endometrium; striae gravidarum (stretch marks); plantar fasciitis; coxarthrosis; muscle rupture; tennis elbow; skin grafting; aging; knee arthritis; burns; renal failure; and atopic diseases, but is not limited thereto.

### 4. Anticancer Agent Comprising a Hydrogel Prepared via ATPS

In one embodiment of the present invention, an anticancer agent comprising a hydrogel prepared according to the above method is provided.

As used herein, the term "anticancer agent" may include a genomics-based anticancer agent or a pharmacogenomics-based anticancer agent. The genomics-based or pharmacogenomics-based anticancer agent refers to an anticancer agent developed by investigating the correlation between a drug and the genome of a biological organism through high-throughput sequencing, bioinformatics, and pharmacology. Specifically, it refers to a genomic variant-customized anticancer agent developed by integrating genomic research into conventional drug development to analyze how genomic variations influence an individual's specific drug response. More specifically, these agents are developed by studying the relationship between various genomic variants or gene expression patterns and pharmacokinetic (PK) factors (the effect of the body on the anticancer agent) or pharmacodynamic (PD) factors (the effect of the chemical substance on the body). Furthermore, the term "genomics-based anticancer agent" also encompasses instances where the focus is on the interaction between an anticancer agent and multiple genes (polygenic) rather than a single gene.

As evident from the foregoing description, genomics-based anticancer agents are founded on the premise that drug efficacy and adverse effects vary according to a patient's genotype. For instance, gefitinib (Iressa^{®}) is known to exhibit superior clinical responses in specific subgroups, such as East Asians, non-smokers, females, and patients diagnosed with adenocarcinoma of non-small cell lung cancer (NSCLC). This high efficacy was subsequently attributed to mutations in the epidermal growth factor receptor (EGFR) gene. Conversely, such EGFR inhibitors are generally ineffective in lung cancers characterized by ALK (anaplastic lymphoma kinase) positivity.

The practice of determining a specific anticancer agent or a combination thereof for a narrow subset of patients harboring particular genetic variants is termed "Precision Medicine." When this scope is narrowed down to a single individual based on their unique genomic profile, it is referred to as "Personalized Medicine." The development of Precision Medicine and genomics-based anticancer agents acknowledges the absence of a "one-size-fits-all" panacea for all cancer patients. It further recognizes that even if broad-spectrum agents exist, they often entail significant adverse effects, much like conventional cytotoxic anticancer agents. Accordingly, the rationale behind these developments is to shift away from the indiscriminate administration of drugs and toward the development of effective therapies that minimize toxicity by specifically considering a patient's genes and their functional roles.

In the present invention, the anticancer agent to be concentrated in the hydrogel may include, without limitation, any drug utilized for the prevention, amelioration, or treatment of cancer. In a preferred embodiment, protein-derived anticancer agents are concentrated within the hydrogel matrix. Specifically, the anticancer agent may be selected from the group consisting of: nitrogen mustard, imatinib, oxaliplatin, rituximab, erlotinib, neratinib, lapatinib, gefitinib, vandetanib, nilotinib, semaxanib, bosutinib, axitinib, cediranib, lestaurtinib, trastuzumab, bortezomib, sunitinib, carboplatin, sorafenib, bevacizumab, cisplatin, cetuximab, Viscum album, asparaginase, tretinoin, hydroxycarbamide, dasatinib, estramustine, gemtuzumab ozogamicin, ibritumomab tiuxetan, heptaplatin, methyl aminolevulinate, amsacrine, alemtuzumab, procarbazine, alprostadil, holmium-166 chitosan complex, gemcitabine, doxifluridine, pemetrexed, tegafur, capecitabine, gimeracil, oteracil, azacitidine, methotrexate, uracil, cytarabine, fluorouracil (5-FU), fludarabine, enocitabine, flutamide, decitabine, mercaptopurine, thioguanine, cladribine, carmofur, raltitrexed, docetaxel, paclitaxel, irinotecan, belotecan, topotecan, vinorelbine, etoposide, vinblastine, idarubicin, mitomycin, bleomycin, dactinomycin, pirarubicin, aclarubicin, peplomycin, temsirolimus, temozolomide, busulfan, ifosfamide, cyclophosphamide, melphalan, altretamine, dacarbazine, thiotepa, nimustine, chlorambucil, mitolactol, leucovorin, exemestane, aminoglutethimide, anagrelide, olaparib, fadrozole, tamoxifen, toremifene, testolactone, anastrozole, letrozole, vorozole, bicalutamide, lomustine, vorinostat, entinostat, and carmustine, but is not limited thereto.

In the present invention, the cancer may be a solid tumor formed by a mass arising from abnormal cell growth in a solid organ. Specific examples include, depending on the location of the solid organ, gastric cancer, liver cancer, glioma, ovarian cancer, colorectal cancer, head and neck cancer, bladder cancer, renal cell carcinoma, breast cancer, metastatic cancer, prostate cancer, pancreatic cancer, melanoma, or lung cancer, among others, but is not limited thereto.

In the present invention, the "prevention" may include, without limitation, any act of blocking symptoms caused by diseases, or suppressing or delaying the symptoms, using the composition of the present invention.

In the present invention, the "treatment" or "amelioration" may include, without limitation, any act capable of ameliorating or beneficially altering symptoms caused by diseases, using the composition of the present invention.

In the present invention, the pharmaceutical composition may be characterized by being in the form of capsules, tablets, granules, injections, ointments, powders, or beverages, and the pharmaceutical composition may be characterized by being targeted to humans.

The pharmaceutical composition of the present invention may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present invention may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, diluents, or excipients suitable for making preparations, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

The route of administration of the pharmaceutical composition according to the present invention includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary depending on a variety of factors, including activity of a certain compound used, the patient's age, body weight, general health status, sex, diet, frequency of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

### 5. Vaccine Comprising a Hydrogel Prepared via ATPS

In one embodiment of the present invention, there is provided a method for preparing a hydrogel, wherein the biological substance of the first aqueous solution is a vaccine.

In another embodiment of the present invention, there is provided a method for preparing a hydrogel, wherein the vaccine is directed against a disease selected from the group consisting of: anthrax, diphtheria, meningitis, hepatitis A, hepatitis B, influenza, Japanese encephalitis, Lyme disease, measles, mumps, pertussis, pneumococcus, polio, rabies, epidemic hemorrhagic fever (hemorrhagic fever with renal syndrome), rotavirus, rubella, herpes zoster, smallpox, tetanus, tuberculosis, typhoid, varicella, Ebola hemorrhagic fever, yellow fever, cholera, human papillomavirus (HPV), and COVID-19 (coronavirus disease 2019).

As used herein, the term "vaccine" encompasses both "prophylactic vaccines" and "therapeutic vaccines." A "prophylactic vaccine" refers to a vaccine intended for the prevention of a disease in healthy individuals, which induces an immune response by administering antigens (e.g., pathogens or portions thereof). A "therapeutic vaccine" refers to a vaccine intended for treating an existing disease in a patient by strengthening the immune system-for instance, through the administration of self-antigens. Such therapeutic vaccines include, but are not limited to, vaccines capable of treating cancer or Alzheimer's disease.

Prophylactic vaccines may be classified according to their production methods into various types, including inactivated vaccines, live-attenuated vaccines, protein subunit vaccines, viral vector-based vaccines, DNA vaccines, and mRNA vaccines.

In addition, the vaccine of the present invention may be formulated as a delayed-release vehicle or a depot preparation. Such long-acting formulations may be administered by implantation (for example, subcutaneously or intramuscularly) or by injection. Thus, for example, the vaccine composition may be formulated with suitable polymeric or hydrophobic materials (for example, as an emulsion in an acceptable oil) or ion exchange resins, or as sparingly soluble derivatives (for example, as a sparingly soluble salt). Liposomes and emulsions are widely known examples of delivery vehicles suitable for use as carriers.

The vaccine of the present invention may also preferably comprise an adjuvant. The adjuvant refers to any substance that, when incorporated into the composition, increases, enhances, or otherwise modifies the immune response induced by said composition. In a broad sense, an adjuvant is defined as a substance that promotes or facilitates an immune response.

The suitable adjuvant includes aluminum salts, such as aluminum hydroxide (*Al(OH)*₃) or aluminum phosphate (*AlPO*₄), but may also be a salt of calcium, iron, or zinc. Furthermore, the adjuvant may be selected from the group consisting of insoluble suspensions of acylated tyrosine or acylated sugars, and cationically or anionically derived saccharides, although it is not limited thereto.

For example, the adjuvant may be selected from the group consisting of: *Al(OH)*₃ or *AlPO*₄, and alum (potassium aluminum sulfate); oil-in-water emulsions such as MF59 or virosomes; Adjuvant Systems (AS) such as AS04 [a mixture of aluminum hydroxide and monophosphoryl lipid A (MPL)], AS03 [a mixture of *DL*-α-tocopherol, squalene, and the emulsifier polysorbate 80], AS01, and AS02; Toll-like receptor (TLR) agonists or pattern recognition receptor agonists such as CpG, flagellin, and Poly I:C; and particulate delivery systems such as ISCOMs (Immune Stimulating Complexes) or ISCOMMATRIX, but is not limited thereto.

In addition, the adjuvant may include cytokines utilized as immunomodulators. For example, cytokines suitable for use as the immunomodulator may be selected from the group consisting of: interleukins (e.g., IL-1, IL-2, IL-4, IL-5, IL-6, IL-7, IL-12, etc.); macrophage colony-stimulating factor (M-CSF); tumor necrosis factor (TNF); and granulocyte-macrophage colony-stimulating factor (GM-CSF), but are not limited thereto.

### 6. Containers Used for Manufacturing Hydrogel

In one embodiment of the present invention, there is provided a container for extracting or concentrating particles, used for manufacturing a hydrogel according to the aforementioned method, the container comprising: a body providing an internal space for accommodating an aqueous solution for ATPS and allowing extraction or concentration of the particles during centrifugation; a first lid provided at one side of the body for injecting blood or a separate substance into the internal space of the body; and a second lid provided at the other side of the body opposite to the first lid for discharging the particles extracted or concentrated in the body.

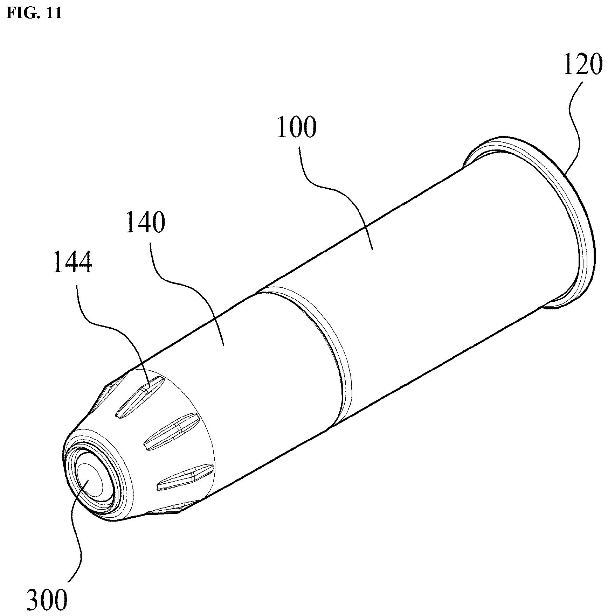

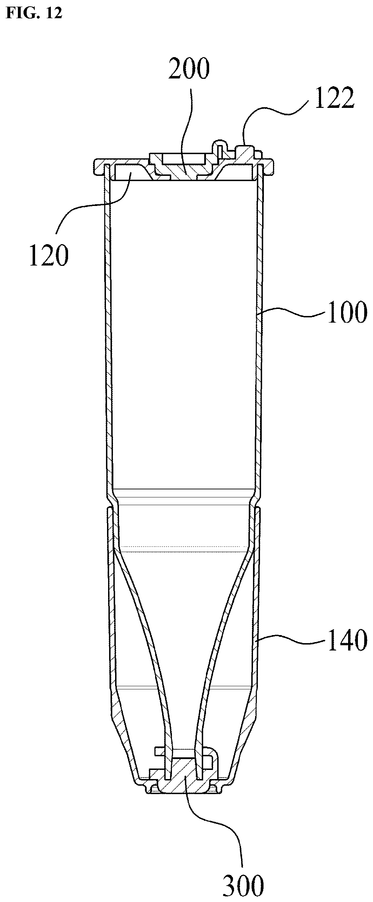

The following is a description of the reference numerals used in FIGS. 11 and 12, which illustrate a container provided in an embodiment of the present invention:
100: Body
120: Cover
122: Fixing member
140: Support
144: Mounting groove
200: First lid
300: Second lid

As used herein, the term "container" is not particularly limited as long as it is a vessel typically used for centrifugation or an Aqueous Two-Phase System (ATPS), and is preferably a medical-grade container. In one embodiment of the present invention, although a container for extracting or concentrating various particles is disclosed, this is merely an exemplary embodiment. The container may be configured to simultaneously extract or concentrate not only various particles but also Regeneration-Rich Plasma (RRP) and platelets. Furthermore, the container may be utilized as a syringe adapter for extracting and concentrating said particles, RRP, and platelets to be applied to the skin or other tissues. Specifically, the body (100) is configured with a converging (tapering) structure toward its lower end. When coupled with a syringe, the container ensures a hermetic seal while featuring a discharge orifice of an appropriate size at the lower end to facilitate the ease of application to the skin. Examples of the medical container include: Syringes: Syringe barrels (hereinafter referred to as "syringes") and prefilled syringes (PFS) pre-loaded with a liquid drug or medicament. As used herein, a "prefilled syringe" refers to a syringe-shaped formulation pre-filled with a liquid drug or medicament, which includes a single-chamber type filled with one type of liquid, and a double-chamber type filled with two types of medicaments. The double-chamber type may include a liquid-powder type (comprising a powder and a solvent) or a liquid-liquid type (comprising two different liquids). Specific examples of syringes used for PFS include pre-fillable syringes, vaccine-specific PFS, anticancer-specific PFS, and needleless syringes. Storage Containers: Wide-mouth bottles, narrow-mouth bottles, medicine bottles, vials, infusion (IV) bottles, bulk containers, Petri dishes, test tubes, and analytical cells. Pharmaceutical & Sampling Vessels: Liquid, powder, or solid drug containers such as ampoules, press-through packages (PTP), infusion bags, and dropper bottles (e.g., ear drops, eye drops); sample containers such as sampling test tubes for blood testing, blood collection tubes, and specimen containers; and analytical vessels such as UV detection cells. Medical & Laboratory Equipment: Sterilization containers for medical instruments such as scalpels, forceps, gauze, or contact lenses; medical devices such as disposable syringes or prefilled syringes; laboratory apparatus such as beakers, vials, ampoules, and flasks; and housings for artificial organs.

As used herein, the "body (100)" provides an internal space for accommodating blood, where various particles are extracted or concentrated during centrifugation. The body (100) is not particularly limited as long as it is suitable for use in centrifugation or an ATPS. In a preferred embodiment, the body (100) is configured to provide an internal space for extracting or concentrating various particles; however, it is not particularly limited thereto, and may provide an internal space for the extraction or concentration of various biological substances in addition to or instead of said particles.

As used herein, the "first lid (200)" is not particularly limited as long as it is capable of sealing the container during the extraction and concentration of various particles, or various particles and platelets, via centrifugation or ATPS. Preferably, the first lid (200) possesses elasticity to enable the hermetic sealing of the container and may be configured as a stopper, plug, or similar member formed of a rubber material.

In another embodiment of the present invention, the first lid (200) provides a container for extracting or concentrating various particles, which is provided on a cover (120) disposed on the upper side of the body (100) to open and close the body.

Furthermore, the "first lid (200)" is openable and closable, and may be separable vertically. A portion of the first lid may be fixed to a fixing member (122). Thus, during the opening/closing process, it can be conveniently opened or closed by being fixed to the fixing member (122). By fixing one side of the first lid to the fixing member, the movement of the first lid during the opening/closing process is restricted, preventing the first lid from separating and being lost. Furthermore, the first lid (200) may be configured to a size suitable for fitting over the body of the syringe. Preferably, it is made of an elastic material, such as rubber, which can seal when fitted over the syringe body.

In the present invention, the "second lid (300)" is openable and closable and may be configured to be a suitable size for fitting the main body of the syringe. Preferably, it is made of an elastic material, such as rubber, which can seal when fitted to the main body of the syringe. Thus, the container used to extract or concentrate various particles according to the above method has a first lid (200) and a second lid (300) at the top and bottom, respectively. This allows a syringe to be inserted either at the top or bottom, providing the advantage of being able to extract any of the vertically separated substances first. Furthermore, when extracting material through the syringe in this case, opening the opposite lid maintains constant pressure inside the device, facilitating easy removal of material with the syringe. Conversely, when only one of the two lids is opened, the pressure balance between the interior of the body (100) and external atmospheric pressure prevents the liquid inside from flowing out. Specifically, when using this container, closing the upper lid prevents liquid inside the body from flowing out downward even if the lower lid is opened.

In another embodiment of the present invention, the opposite side of the body (100) is formed to taper toward the second lid (300), providing a container for extracting or concentrating various particles. Thus, forming the other side of the body (100) to taper toward the second lid (300) provides the advantage that even a small amount of biological material can be withdrawn through the bottom using a syringe. Furthermore, when used as a syringe adapter for application to skin or the like, even a small amount of biological material can be applied accurately. Furthermore, for this purpose, graduations indicating volume may be marked on the body (100).

In another embodiment of the present invention, a container for extracting or concentrating various particles and RRP is provided, further including a support body (140) that wraps around the other outer surface of the body (100) around the second lid (300). As previously noted, the other side of the body (100) is formed to taper toward the second lid (300), which may present disadvantages such as instability during centrifugation, leading to breakage, or requiring storage in a horizontal position. However, by further including the support (140), it provides the advantage of sturdily supporting the body (100) even during centrifugation and improving storage convenience by enabling upright storage.

Furthermore, to enable stable mounting of containers for extracting or concentrating various particles and RRP according to the present invention onto centrifugal devices, mounting grooves (144) may be provided on the outer surface of the support (140).

In one embodiment of the present invention, a method for manufacturing a hydrogel is provided, comprising: 1) mixing a first aqueous solution and a second aqueous solution under cationic salt conditions to form an aqueous two-phase system; and 2) forming a porous hydrogel structure in one of the phases through the aqueous two-phase system.

In another embodiment of the present invention, a method for manufacturing a hydrogel is provided, further comprising the step of: 3) forming a hydrogel containing biological material within the porous hydrogel structure.

In yet another embodiment of the present invention, the solute in the first aqueous solution is hyaluronic acid, collagen, chitosan, dextran (DEX), alginate, carboxymethylcellulose (CMC), polyacrylamide (PAM), or polyvinyl alcohol (PVA).

In another embodiment of the present invention, the solute in the second aqueous solution is selected from the group consisting of polyethylene glycol (PEG), propylene glycol (PPG), polyvinyl pyrrolidone (PVP), polyvinyl alcohol(polyvinyl alcohol, PVOH, PVA, PVAI), PVME (poly(vinyl methyl ether)), HP-Starch, UCON, polyethersulfone (PES), pullulan, PPGDME (Polypropylene Glycol Dimethyl Ether), dextran (DEX), maltodextrin, hydroxyethylcellulose (Natrosol), PEGME (Polyethylene Glycol Methyl Ether), PEGDME (Polyethylene Glycol Dimethyl Ether), Polyacrylamide (PAM), PSS (Poly(styrene sulfonate)), DEAE-Dextran, Polyethylene Imine (PEI), Hydroxypropyl Methylcellulose (HPMC), PNIPAM (Poly(N-isopropylacrylamide)), PDADMAC (Poly(diallyldimethylammonium chloride)), PVCL (Polyvinyl Caprolactam), and Ficoll.

In another embodiment of the present invention, a method for manufacturing a hydrogel is provided wherein the mass percentage of the solute in the first aqueous solution is 0.1 to 5% (w/w).

In another embodiment of the present invention, a method for manufacturing a hydrogel is provided wherein the average molecular weight of the solute in the first aqueous solution is 1,000 Da or more and 500,000 Da or less.

In another embodiment of the present invention, a method for manufacturing a hydrogel is provided wherein the solute in the first aqueous solution has an average molecular weight of 10 to 50,000 kDa.

Another embodiment of the present invention provides a method for manufacturing a hydrogel wherein the concentration of the solute in the first aqueous solution is 0.16 wt% or more.

Another embodiment of the present invention provides a method for manufacturing a hydrogel wherein the mass percentage of the polyethylene glycol (PEG) is 3 to 50% (w/w).

In another embodiment of the present invention, a method for manufacturing a hydrogel is provided, wherein the ratio of the average molecular weight (kDa) of the solute in the first aqueous solution to the mass percentage (w/w) of the solute in the second aqueous solution is 5 kDa/(w/w) to 1,000 kDa/(w/w). In another embodiment of the present invention, the ratio of the average molecular weight (kDa) of the solute in the first aqueous solution to the mass percentage (w/w) of the solute in the second aqueous solution may be from a minimum of 1 kDa/(w/w) to a maximum of 1,000 kDa/(w/w), and may be 1 to 1,000 kDa/(w/w), 3 to 1,000 kDa/(w/w), 1 to 900 kDa/(w/w), 1 to 800 kDa/(w/w), 1 to 700 kDa/(w/w), 1-600 kDa/(w/w), 1-500 kDa/(w/w), 1-400 kDa/(w/w), 1-300 kDa/(w/w), 1-200 kDa/(w/w), 1-100 kDa/(w/w), 3-900 kDa/(w/w), 3-800 kDa/(w/w), 3-700 kDa/(w/w), 3~600 kDa/(w/w), 3~500 kDa/(w/w), 3~400 kDa/(w/w), 3~300 kDa/(w/w), 3~200 kDa/(w/w), 3~100 kDa/(w/w), but is not limited thereto.

In another embodiment of the present invention, a method for manufacturing a hydrogel is provided wherein the cationic salt is selected from the group consisting of monovalent cations, divalent cations, or trivalent cations.

In another embodiment of the present invention, a method for manufacturing a hydrogel is provided wherein the mass percentage of the cationic salt is 0.5 to 20% (w/w).

In another embodiment of the present invention, a method for manufacturing a hydrogel is provided wherein the monovalent cation is selected from the group consisting of Na+, K+, or Ag+.

Another embodiment of the present invention provides a method for manufacturing a hydrogel wherein the divalent cation is selected from the group consisting of Ca²⁺, Mg²⁺, Ba²⁺, or Pb²⁺.

Another embodiment of the present invention provides a method for manufacturing a hydrogel wherein the trivalent cation is an Al³⁺ cation.

Another embodiment of the present invention provides a method for manufacturing a hydrogel wherein the biological material is mesenchymal stem cells (MSC), MSC-derived Exosome, virus, protein, RNA, Platelet-Rich Plasma (PRP), or regeneration factor.

In another embodiment of the present invention, the regeneration factor is selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

In another embodiment of the present invention, the protein is a protein-derived anticancer agent, providing a method for manufacturing a hydrogel.

In another embodiment of the present invention, the biological material is a genome-based anticancer agent, and the method of manufacturing a hydrogel is provided.

In another embodiment of the present invention, the biological material is a vaccine, and a method for manufacturing a hydrogel is provided.

In another embodiment of the present invention, the vaccine is selected from the group consisting of anthrax, diphtheria, meningitis, hepatitis A, hepatitis B, influenza, Japanese encephalitis, Lyme disease, measles, mumps, pertussis, pneumococcal disease, polio, rabies, hemorrhagic fever with renal syndrome, rotavirus, rubella, shingles, smallpox, tetanus, tuberculosis, typhoid fever, chickenpox, Ebola hemorrhagic fever, yellow fever, cholera, human papillomavirus, and coronavirus disease-19.

In another embodiment of the present invention, the first aqueous solution provides a method for manufacturing a hydrogel wherein it acts as a backbone in hydrogel formation.

In one embodiment of the present invention, a hydrogel manufactured according to the above manufacturing method is provided.

In one embodiment of the present invention, a skin beautification method is provided, comprising administering the hydrogel manufactured according to the manufacturing method to a subject.

In one embodiment of the present invention, a skin cosmetic composition is provided, comprising administering the hydrogel manufactured according to the manufacturing method to a subject.

In one embodiment of the present invention, a cosmetic containing the aforementioned skin beautifying composition is provided.

In one embodiment of the present invention, a wound treatment composition containing the hydrogel manufactured according to the above manufacturing method is provided.

Another embodiment of the present invention provides a wound treatment composition containing the hydrogel manufactured according to the aforementioned manufacturing method.

In another embodiment of the present invention provides a composition for treating a wound, wherein the wound is selected from the group consisting of an open wound, pressure sore, burn, abrasion, puncture ulcerative wound, stab wound, chronic skin wound caused by reactive oxygen species, contusion, cut wound, wound of the throat or oral mucosa, lacerations, diabetic ulcers, leg ulcers, hypertensive ischemic ulcers, venous ulcers, and foot ulcers.

In one embodiment of the present invention, a regenerative composition containing a hydrogel manufactured according to the above manufacturing method is provided.

In another embodiment of the present invention, the regeneration is for the treatment of maxillofacial disorders, gastric ulcers treated by endoscopic submucosal dissection, human autologous fat grafting, knee osteoarthritis, chronic non-healing ulcers, hair regeneration therapy for male pattern baldness, Achilles tendon rupture, high-risk proliferative diabetic retinopathy, knee OA, patellar dislocation, lumbar facet joint syndrome, pigmentary retinopathy, diabetic macular edema, traumatic bone cysts, high-risk proliferative diabetic retinopathy, scars, dry eye syndrome, Sjögren's syndrome, Osteoarthritis, Meniscal Lesions, Melasma, Muscle Injury, Ulnar Nerve Neuropathy of the Elbow, Chronic Ulcers, Alopecia, Supracondylar Fracture, Bone Resorption, Rotator Cuff Tear, Thin Endometrium, Pregnancy Line, Plantar Fasciitis, Hip Osteoarthritis, Osteoarthritis, muscle rupture, tennis elbow, skin grafting, aging, knee arthritis, burns, renal failure, and atopic disease.

In one embodiment of the present invention, a container used to manufacture a hydrogel produced according to the manufacturing method comprises: a body that accommodates an aqueous solution for the ATPS and provides an internal space where particles are extracted or concentrated during centrifugation; a first lid provided on one side of the body to inject blood or a separate substance into the internal space of the body; and a second lid provided on the opposite side of the body to discharge the particles extracted or concentrated from the body.

In another embodiment of the present invention, the particles are selected from the group consisting of mesenchymal stem cells (MSC), MSC-derived exosomes, viruses, proteins, RNA, and regeneration factors, and the container extracts or concentrates one or more of these particles.

In yet another embodiment of the present invention, the regeneration factor is selected from the group consisting of bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), platelet-derived growth factor (PDGF), placental growth factor (PlGF), and hepatocyte growth factor (HGF).

In another embodiment of the present invention, the first lid provides a container for extracting or concentrating particles, which is provided on a cover disposed on the upper side of the body to open and close the body.

In another embodiment of the present invention, a container for extracting or concentrating particles is provided wherein the opposite side of the body is formed to narrow toward the second lid.

In another embodiment of the present invention, a container for extracting or concentrating particles is provided, further including a support that wraps around the outer surface of the other side of the body around the second lid.

### Advantageous Effects

When biological materials sensitive to external reactions are included during hydrogel formation, the physical or chemical stimuli applied in conventional hydrogel manufacturing methods require sophisticated processes and may affect the activity and structure of particles within the hydrogel. In contrast, the hydrogel and its manufacturing method using an aqueous two-phase system (ATPS) provided by the present invention simultaneously enable hydrogel formation and encapsulation of desired nanoparticles within the hydrogel simultaneously. This enables the simple concentration of desired nanoparticles within the hydrogel at high yield. Furthermore, since concentration and hydrogel formation occur concurrently, the hydrogel manufacturing time is shortened and costs are significantly reduced.

### Brief Description of Drawings

FIGS. 1 and 2 are schematic diagrams illustrating a hydrogel and a method for manufacturing the same using an aqueous two-phase system (ATPS) according to the present invention.
FIG. 3 is an image confirming the simultaneous occurrence of particle separation and hydrogel formation via the ATPS of the present invention; specifically, it illustrates that a hydrogel formed in a bottom phase of the ATPS has absorbed particles dyed in red, as visually confirmed by the red color of the resulting hydrogel.
FIGS. 4a to 4e are charts illustrating the formation of hyaluronic acid hydrogels.
FIG. 5 shows binodal curve data obtained from experiments performed while varying the molecular weight of sodium hyaluronate (hyaluronic acid).
FIG. 6 shows experimental data on hydrogel formation performed by varying the type of ions among sodium (*Na*⁺), calcium (*Ca*2⁺), and magnesium (*Mg*²⁺).
FIG. 7a is a graph illustrating the recovery efficiency for each particle, wherein the recovery efficiency refers to the ratio of the recovered particles to the initial particles.
FIG. 7b is a graph illustrating the concentration ratio for each particle, wherein the concentration ratio refers to the ratio of the concentration of the recovered particles to the initial concentration of the particles.
FIG. 8a shows the results confirming the change in diffusion rate depending on the presence or absence of hydrogel encapsulation using a food-grade red dye.
FIG. 8b shows the results confirming the release profile of the encapsulated red dye.
FIG. 9 shows toxicity test results, specifically illustrating a comparison of body weights against a control group, wherein G1 denotes the control group and G2 denotes the group administered with the encapsulated hydrogel formed via the ATPS of the present invention.
FIG. 10 shows results confirming the enhancement in efficacy of the encapsulated hydrogel formed via the ATPS of the present invention.
FIG. 11 is a perspective view illustrating an embodiment of a container for extracting or concentrating particles according to the present invention.
FIG. 12 is a cross-sectional view of the container for extracting or concentrating particles illustrated in FIG. 11.
FIG. 13 is a chart illustrating hydrogel formation within the ATPS according to the present invention.

### Mode for Invention

Hereinafter, the present invention will be described in more detail by way of examples. These examples are only for illustrating the present invention in more detail, and it will be obvious to those skilled in the art that the scope of the present invention according to the subject matter of the present invention is not limited by these examples.

### [Examples]

### [Example 1] Overview of the Invention

An aqueous two-phase system (ATPS) is a method for concentrating and extracting particles by forming two distinct aqueous phases. To effectively concentrate and extract target substances, the types and concentrations of the materials utilized to form the ATPS are critical. In particular, by adjusting the types and concentrations of the materials constituting the ATPS according to specific objectives, hydrogel formation may be induced under particular conditions. This allows for the simultaneous occurrence of particle separation via the ATPS and the formation of the hydrogel matrix.

In a case where particle separation via the ATPS and hydrogel formation occur simultaneously, hyaluronic acid (HA) is concentrated into one of the phases to form an aqueous solution for a sol-gel process. Concurrently, other particles are partitioned and concentrated into one of the two aqueous phases in accordance with the inherent separation characteristics of the ATPS. The hyaluronic acid aqueous phase undergoing the sol-gel process precipitates due to density differences, and the precipitation of the HA phase and the subsequent hydrogel formation can be accelerated through centrifugation. Specifically, during centrifugation, the cross-linking of the hyaluronic acid may be accelerated by the action of gravity and centrifugal force.

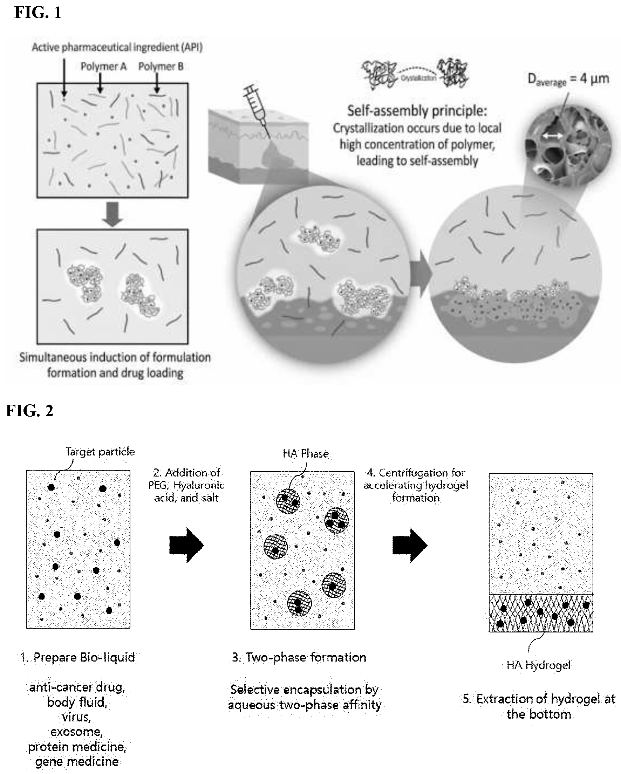

A general overview of the present invention described in this embodiment is schematically illustrated in FIGS. 1 and 2.

### [Example 2] Verification of Simultaneous Particle Separation and Encapsulation Hydrogel Formation

Red-dyed particles (Red beads) with a diameter of 500 nm were mixed with water, followed by hyaluronic acid (HA) encapsulation. As a result, it was confirmed that an aqueous HA phase undergoing a sol-gel process was formed, which absorbed the red-dyed particles and precipitated into the bottom phase of the ATPS.

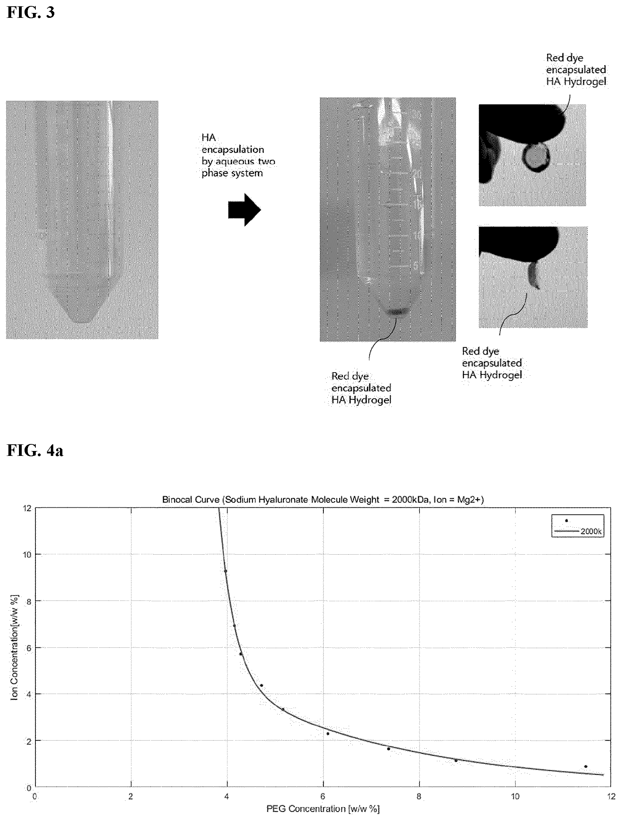

Furthermore, as shown in FIG. 3, when the aqueous HA phase in the sol-gel process was centrifuged to finally form a hydrogel in a gel state, and the resulting hydrogel was separated and removed from the solution, it was observed that the hydrogel maintained its shape. It was further confirmed that the red-dyed particles (Red beads) were concentrated within the precipitated hydrogel.

### [Example 3] Optimization of Materials and Composition for Aqueous Solution Bifurcation Method to Form Hydrogel

Optimization of the materials and compositions for forming the encapsulated hydrogel prepared in Example 2 was performed. Hydrogel formation utilizing an ATPS occurs at specific concentration compositions and is dependent on the concentration or molecular weight of polyethylene glycol (PEG), salts, and hyaluronic acid (HA). Accordingly, through repeated experiments, the *PEG* concentration (*w*/*w*%), the type of ions, and the molecular weight of HA required for successful hydrogel formation were characterized.

### [Example 3-1] Encapsulation Hydrogel Formation 1 (Mg²⁺ Ion, Hyaluronic Acid Molecular Weight 2,000 kDa)

To evaluate the conditions for hydrogel formation using an ATPS, the type of ions was fixed to magnesium ions (*Mg*²⁺) and the molecular weight of hyaluronic acid (HA) was fixed to 2,000 kDa. While varying the concentrations (*w*/*w*%) of polyethylene glycol (PEG) and the ions, the formation of the hydrogel was observed, and the results are presented as a binodal curve graph in FIG. 4a. Specifically, in the graph, the region above the dotted line represents the compositions where the hydrogel is formed, whereas the region below the dotted line represents the compositions where the hydrogel is not formed.

As a result, as shown in FIG. 4a, it was confirmed that the hydrogel according to the ATPS was formed when the ion concentration (*w*/*w*%) was approximately 1% or higher and the PEG concentration (*w*/*w*%) was in the range of approximately 4% to 12%. More specifically, when the average molecular weight of the solute in the first aqueous solution (hyaluronic acid) was 2,000 kDa, the mass percentage (*w*/*w*%) of the solute in the second aqueous solution (PEG) ranged from 3 *w*/*w*% to 15 *w*/*w*%. Under these conditions, it was confirmed that the ratio of the average molecular weight (kDa) of the solute in the first aqueous solution to the mass percentage (*w*/*w*) of the solute in the second aqueous solution was in the range of 100 to 1,000 *kDa*(*w*/*w*).

### [Example 3-2] Encapsulation Hydrogel Formation 2 (Mg²⁺ Ion, Hyaluronic Acid Molecular Weight 200 kDa)

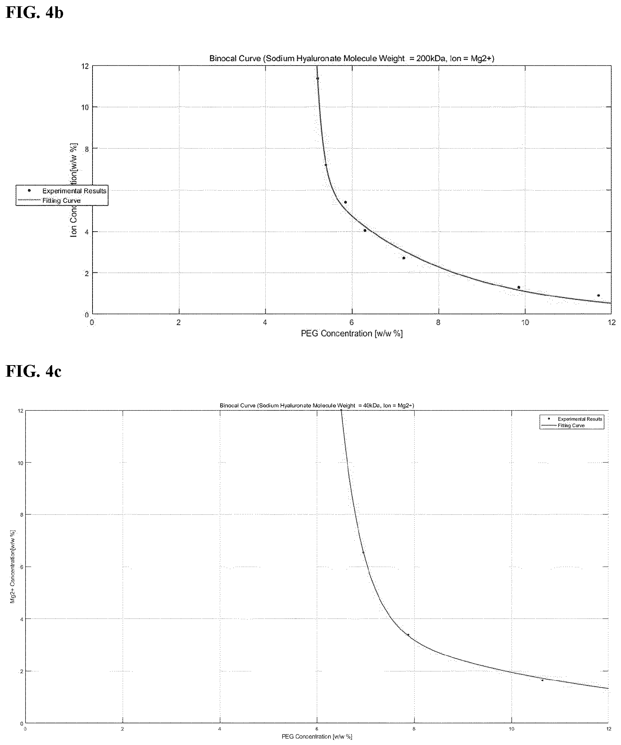

To evaluate the conditions for hydrogel formation using an ATPS, the type of ions was fixed to magnesium ions (*Mg*²⁺) and the molecular weight of hyaluronic acid (HA) was fixed to 200 kDa. While varying the concentrations (*w*/*w*%) of polyethylene glycol (PEG) and the ions, the formation of the hydrogel was observed, and the results are presented as a binodal curve graph in FIG. 4b. Specifically, in the graph, the region above the dotted line represents the compositions where the hydrogel is formed, whereas the region below the dotted line represents the compositions where the hydrogel is not formed.

As a result, as shown in FIG. 4b, it was confirmed that the hydrogel according to the ATPS was formed when the ion concentration (*w*/*w*%) was approximately 1% or higher and the PEG concentration (*w*/*w*%) was in the range of approximately 5% to 12%. More specifically, when the average molecular weight of the solute in the first aqueous solution (hyaluronic acid) was 200 kDa, the mass percentage (*w*/*w*%) of the solute in the second aqueous solution (PEG) ranged from 4 *w*/*w*% to 15 *w*/*w*%. Under these conditions, it was confirmed that the ratio of the average molecular weight (kDa) of the solute in the first aqueous solution to the mass percentage (*w*/*w*%) of the solute in the second aqueous solution was in the range of 10 to 50 *kDa*(*w*/*w*).

### [Example 3-3] Encapsulation Hydrogel Formation 3 (Mg²⁺ Ion, Hyaluronic Acid Molecular Weight 40 kDa)

To evaluate the conditions for hydrogel formation using an aqueous two-phase system (ATPS), the type of ions was fixed to magnesium ions (*Mg*²⁺) and the molecular weight of hyaluronic acid (HA) was fixed to 40 kDa. While varying the concentrations (*w*/*w*%) of polyethylene glycol (PEG) and the ions, the formation of the hydrogel was observed, and the results are presented as a binodal curve graph in FIG. 4c. Specifically, in the graph, the region above the dotted line represents the compositions where the hydrogel is formed, whereas the region below the dotted line represents the compositions where the hydrogel is not formed.

As a result, as shown in FIG. 4c, it was confirmed that the hydrogel according to the ATPS was formed when the ion concentration (*w*/*w*%) was approximately 1% or higher and the PEG concentration (*w*/*w*%) was in the range of approximately 6% to 12%. More specifically, when the average molecular weight of the solute in the first aqueous solution (hyaluronic acid) was 40 kDa, the mass percentage (*w*/*w*%) of the solute in the second aqueous solution (PEG) ranged from 5 *w*/*w*% to 20 *w*/*w*%. Under these conditions, it was confirmed that the ratio of the average molecular weight (kDa) of the solute in the first aqueous solution to the mass percentage (*w*/*w*%) of the solute in the second aqueous solution was in the range of 2 to 8 *kDa*(*w*/*w*).

### [Example 3-4] Encapsulation Hydrogel Formation 4 (Ca²⁺ Ion, Hyaluronic Acid Molecular Weight 2,000 kDa)

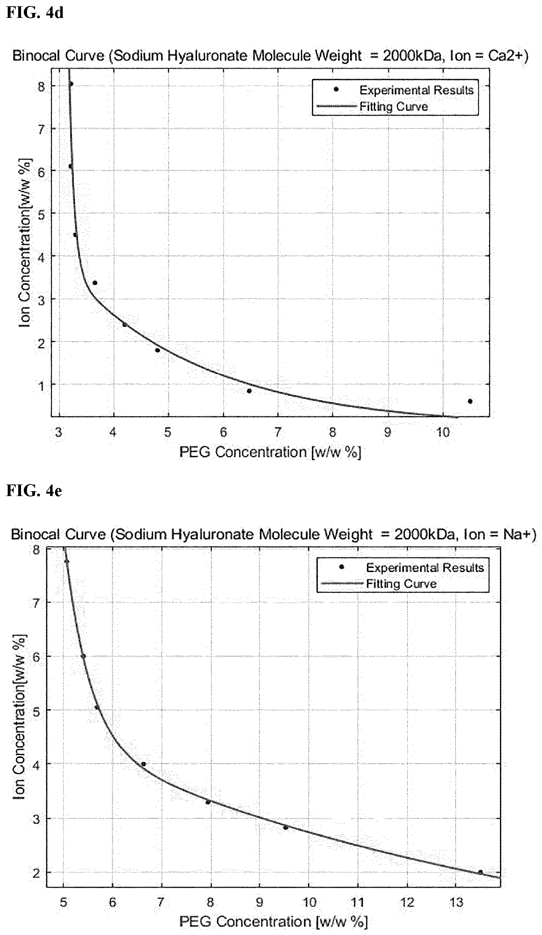

To evaluate the conditions for hydrogel formation using an ATPS, the type of ions was fixed to calcium ions (*Ca*²⁺) and the molecular weight of hyaluronic acid (HA) was fixed to 2,000 kDa. While varying the concentrations (*w*/*w*%) of polyethylene glycol (PEG) and the ions, the formation of the hydrogel was observed, and the results are presented as a binodal curve graph in FIG. 4d. Specifically, in the graph, the region above the dotted line represents the compositions where the hydrogel is formed, whereas the region below the dotted line represents the compositions where the hydrogel is not formed.

As a result, as shown in FIG. 4d, it was confirmed that the hydrogel according to the ATPS was formed when the ion concentration (*w*/*w*%) was approximately 1% or higher and the PEG concentration (*w*/*w*%) was in the range of approximately 3% to 10%. More specifically, when the average molecular weight of the solute in the first aqueous solution (hyaluronic acid) was 2,000 kDa, the mass percentage (*w*/*w*) of the solute in the second aqueous solution (PEG) ranged from 3 *w*/*w*% to 10 *w*/*w*%. Under these conditions, it was confirmed that the ratio of the average molecular weight (kDa) of the solute in the first aqueous solution to the mass percentage (*w*/*w*%) of the solute in the second aqueous solution was in the range of 200 to 700 *kDa*(*w*/*w*).

### [Examples 3-5] Encapsulation Hydrogel Formation 4 (Na⁺ Ion, Hyaluronic Acid Molecular Weight 2,000 kDa)

To evaluate the conditions for hydrogel formation using an aqueous two-phase system (ATPS), the type of ions was fixed to sodium ions (*Na*⁺) and the molecular weight of hyaluronic acid (HA) was fixed to 2,000 kDa. While varying the concentrations (*w*/*w*%) of polyethylene glycol (PEG) and the ions, the formation of the hydrogel was observed, and the results are presented as a binodal curve graph in FIG. 4e. Specifically, in the graph, the region above the dotted line represents the compositions where the hydrogel is formed, whereas the region below the dotted line represents the compositions where the hydrogel is not formed.

As a result, as shown in FIG. 4e, it was confirmed that the hydrogel according to the ATPS was formed when the ion concentration (*w*/*w*%) was approximately 2% or higher and the PEG concentration (*w*/*w*%) was in the range of approximately 5% to 13%. More specifically, when the average molecular weight of the solute in the first aqueous solution (hyaluronic acid) was 2,000 kDa, the mass percentage (*w*/*w*%) of the solute in the second aqueous solution (PEG) ranged from 5 *w*/*w*% to 15 *w*/*w*%. Under these conditions, it was confirmed that the ratio of the average molecular weight (kDa) of the solute in the first aqueous solution to the mass percentage (*w*/*w*%) of the solute in the second aqueous solution was in the range of 100 to 400 *kDa*(*w*/*w*).

Analyzing the graphs in FIGS. 4a to 4e, it can be observed that the position of the curve for hydrogel formation shifts depending on the molecular weight of the hyaluronic acid or when utilizing materials other than hyaluronic acid (e.g., chitosan, collagen, or dextran). According to two-phase theory, it is estimated that as materials with higher molecular weights and higher hydrophilicity are utilized, the curve will shift toward the lower-left region of the graph.

### [Example 4] Confirmation of Hydrogel Formation via ATPS According to the Composition Ratio of Cations and PEG

Next, experiments were conducted to form hydrogels through an ATPS, and binodal curve data were secured. The primary objective of this experiment was to identify the boundary of hydrogel formation based on the composition ratio of magnesium ions (*Mg*²⁺) and PEG and to observe the effects of varying the concentration of sodium hyaluronate.

The materials utilized in the experiment were a 45 wt/wt% PEG stock solution, a 1 wt/wt% sodium hyaluronate stock solution, and a 45 wt/wt% magnesium ion stock solution. During the hydrogel formation process, it was confirmed that the hydrogel formed immediately upon mixing the PEG, magnesium ions, distilled water, and sodium hyaluronate. Initially, a uniform hydrogel was formed, and over time, a phenomenon of localized aggregation was observed.

To construct the binodal curve, the opacity of the solution was checked immediately after mixing the additives, and the visual formation of the hydrogel was verified through high concentrations of additives. Subsequently, additional distilled water and sodium hyaluronate were added to adjust the concentrations of PEG and magnesium ions while maintaining the sodium hyaluronate concentration of the sample. During this process, the final point at which the hydrogel structure was maintained was identified. The point where the hydrogel failed to form or maintain its structure and disappeared when diluted below specific concentrations of PEG and magnesium ions was observed, and the binodal curve was constructed based on this information.

As a result, as illustrated in FIGS. 4a to 4e, the ratio of the mass percentage of cations (wt/wt%) to the mass percentage of polyethylene glycol (wt/wt%) was found to be in the range of 1/50 to 5.

### [Example 5] Confirmation of Hydrogel Formation via ATPS According to Hyaluronic Acid Molecular Weight

Subsequently, experiments were conducted by varying the molecular weight of sodium hyaluronate, and the tendencies observed in the resulting binodal curves were analyzed. The boundaries for hydrogel formation based on the combination of magnesium ions (*Mg*²⁺, wt%) and PEG (wt%) were identified in the graph.

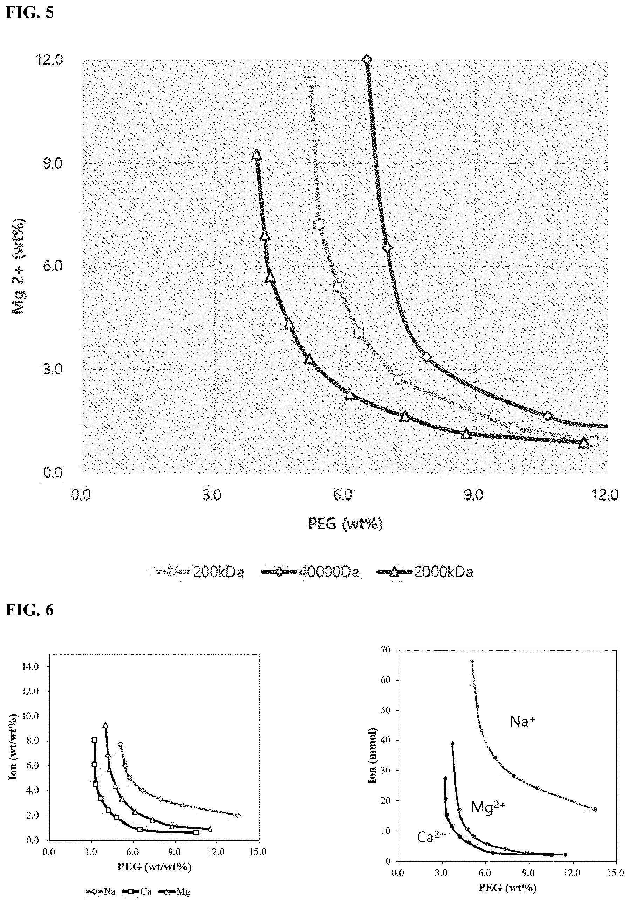

According to the results illustrated in the graph of FIG. 5, a trend is observed where the concentration of PEG required for hydrogel formation decreases as the molecular weight of sodium hyaluronate increases. Specifically, starting from the lowest molecular weight of 40,000 Da and increasing to 200 kDa and 2,000 kDa, it was confirmed that the PEG concentration required for hydrogel formation becomes progressively lower. This is attributed to the fact that higher molecular weight sodium hyaluronate more effectively facilitates hydrogel formation.

Consequently, as the molecular weight of sodium hyaluronate increases, the minimum conditions required for hydrogel formation through the combination of PEG and *Mg*²⁺ tend to become less stringent.

### [Example 6] Confirmation of Hydrogel Formation via ATPS According to Cation Type

Hydrogel formation experiments were conducted by varying the type of ions among sodium (*Na*⁺), calcium (*Ca*²⁺), and magnesium (*Mg*²⁺). The objective of this experiment was to analyze the changes according to the charge and ionic strength of each ion and to characterize the resulting shifts in the binodal curve.

As illustrated in FIG. 6, the position of the binodal curve shifts depending on the type of ions added. Since *Na*⁺, *Mg*²⁺, and *Ca*²⁺ ions exhibit different charges and ionic strengths, they influence the optimal PEG concentration required for hydrogel formation. *Na*⁺, a monovalent cation, exhibits relatively low reactivity with sodium hyaluronate; therefore, a higher concentration of additives is required for hydrogel formation compared to cases utilizing multivalent ions.

Due to its lower charge, *Na*⁺ tends to result in a binodal curve formed at relatively higher PEG concentrations. This indicates that the influence of *Na*⁺ on the hydrogel structure is limited. In contrast, *Mg*²⁺ and *Ca*²⁺ each carry two positive charges, causing the binodal curve to form at lower PEG concentrations. This trend suggests that *Mg*²⁺ and *Ca*²⁺ contribute more significantly to the facilitation of hydrogel formation.

In conclusion, the phenomenon wherein the position of the binodal curve shifts according to the charge and ionic strength of the ions reflects the interaction strength of the ions and their subsequent impact on hydrogel formation.

### [Example 7] Confirmation of Hyaluronic Acid Encapsulation (HA encapsulation)

Experiments were conducted to verify the encapsulation of various biological substances within hyaluronic acid (HA), utilizing a molecular weight of 2,000 kDa and magnesium ions (*Mg*²⁺). Specifically, HA encapsulation was applied to mesenchymal stem cells (MSCs), MSC-derived exosomes, viruses, proteins, and RNA to evaluate the feasibility of the encapsulation process.

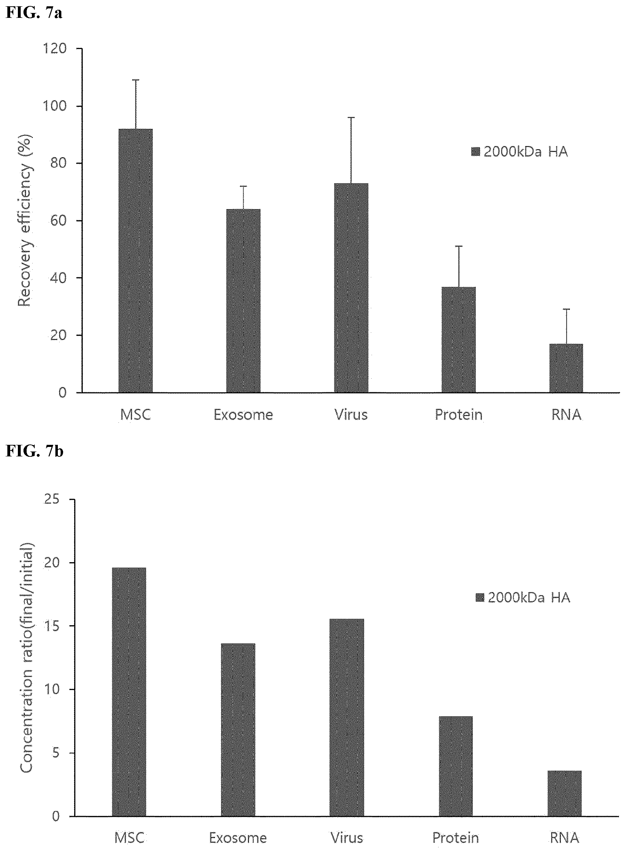

As illustrated in FIGS. 7a and 7b, it was confirmed that HA encapsulation was successfully achieved for MSCs, exosomes, viruses, proteins, and RNA, resulting in the formation of hydrogels containing each respective particle. Most particles exhibited a recovery efficiency ranging from 40% to over 90%, demonstrating that the hydrogel formed via the ATPS of the present invention is capable of concentrating and retaining these particles at high densities. In the case of RNA, although the recovery efficiency was relatively lower than that of other particles, it still exceeded 20%. This difference is expected to be attributed to the specific affinity of RNA for the hyaluronic acid aqueous phase.

Furthermore, the partitioning of these particles can be optimized for specific target substances by modifying the type of salts or by incorporating additives in accordance with two-phase theory.

### [Example 8-1] Confirmation of Release Characteristics of the Hydrogel

To evaluate the efficacy of the hydrogel as a sustained-release system, changes in diffusion rates based on the presence or absence of hydrogel encapsulation were investigated using a food-grade red dye. The concentration and quantity of the red dye added in the experiment were set to be identical for all samples.

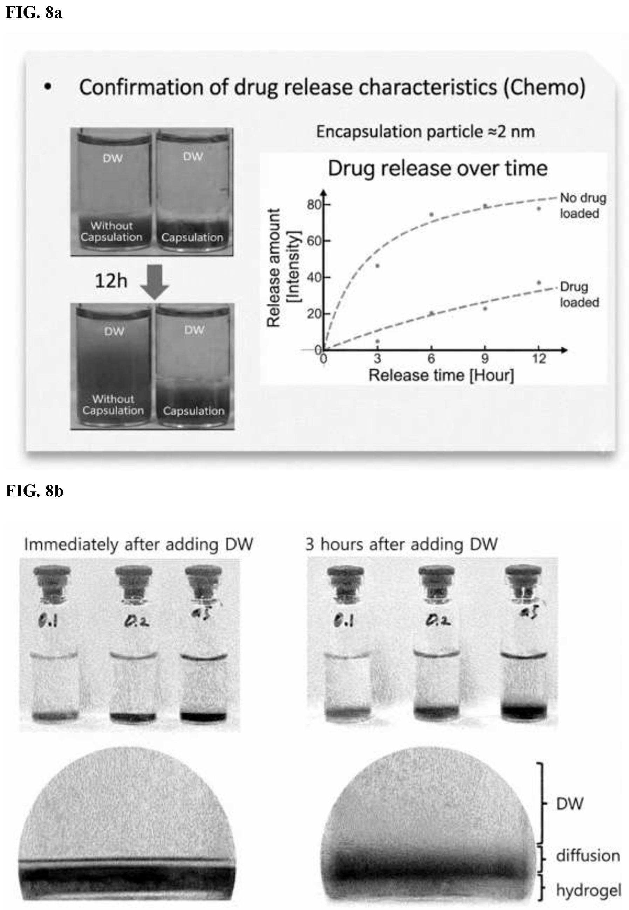

As illustrated in FIG. 8a, a comparison of the diffusion tendencies of the red dye revealed that the diffusion rate of the encapsulated sample was significantly slower than that of the non-encapsulated sample. These results demonstrate that the release rate of particles can be effectively modulated through encapsulation within the hydrogel matrix.

Experimental results indicated a clear trend of delayed release of the red dye in the encapsulated hydrogel, signifying that the mass transfer mechanism within the hydrogel is effectively hindered by the encapsulation. This study provides a crucial foundation for the development of drug delivery systems and the tuning of the physical properties of hydrogels.

### [Example 8-2] Confirmation of Release Characteristics of the Hydrogel

Subsequently, encapsulation within the hydrogel was induced using a food-grade red dye. After providing a gelation time of 3 hours, distilled water was added to observe the release profile of the encapsulated red dye.

As illustrated in FIG. 8b, upon adding distilled water to the red dyeencapsulated hydrogel and performing follow-up observations over time, a phenomenon was observed wherein the dye particles migrated from the interface between the hydrogel and the distilled water into the distilled water layer as time progressed.

### [Example 9] Confirmation of Acute Systemic Toxicity of the Hydrogel

Subsequently, the systemic toxicity of the hydrogel prepared via the ATPS in the aforementioned examples was evaluated in ICR mice following acute intraperitoneal administration.

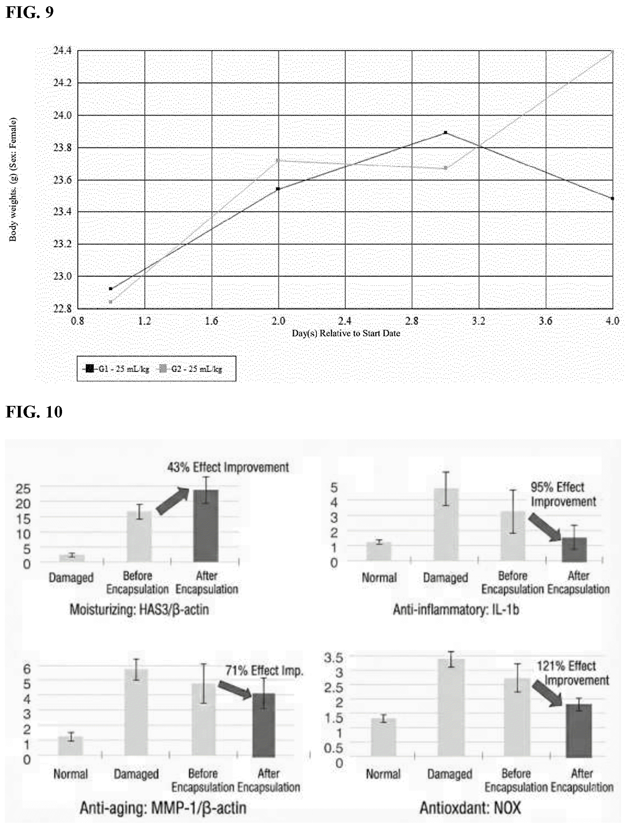

The test group was administered the hydrogel at a dose level of 25 mL/kg, while a control group was established and administered only physiological saline injections. Each group consisted of three female mice. As illustrated in FIG. 9, the toxicity assessment included mortality, clinical signs, body weight changes, and necropsy findings. Upon comparing the test group with the control group, no toxicity associated with the hydrogel was observed in terms of mortality, clinical signs, body weight, or necropsy findings.

### [Example 10] Confirmation of Efficacy Enhancement of the Sustained-Release Hydrogel

Subsequently, to evaluate the effectiveness of the hydrogel prepared via the ATPS in the aforementioned examples as a sustained-release system, biological substances encapsulated within the hydrogel were treated and analyzed in vitro. Specifically, the effectiveness of the sustained-release hydrogel was verified by monitoring *HAS3*/*beta-actin* for enhanced moisturizing effects, *IL-1beta* for enhanced anti-inflammatory effects, *MMP-1*/*beta-actin* for enhanced anti-aging effects, and *NOX* for antioxidant effects.

As illustrated in FIG. 10, an efficacy enhancement of up to 121% was confirmed across each indicator (moisturizing, anti-inflammatory, anti-aging, and antioxidant). It was confirmed that the cause of this efficacy enhancement is that the drugs are delivered to the cells more continuously and for a longer duration when applied onto the cells while encapsulated within the porous hydrogel, as compared to when the drugs are treated alone.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A method of preparing a hydrogel, comprising:
mixing a first aqueous solution and a second aqueous solution in the presence of a cationic salt to form an aqueous two-phase system; and
forming a porous hydrogel structure in one of the phases of the aqueous two-phase system.

2. The method of claim 1, further comprising:
forming a hydrogel comprising a biological material in the porous hydrogel structure.

3. The method of claim 2, wherein the solute of the first aqueous solution is selected from the group consisting of:
a natural polymer group consisting of hyaluronic acid, gelatin, alginate, xanthan gum, chitosan, pectin, galactomannan, glucomannan, fibrin, collagen, dextran (DEX), carboxymethyl cellulose (CMC), agar, psyllium husk, methylcellulose, poly(lysine), and dextran succinic acid; and
a synthetic polymer group consisting of polyacrylamide (PAM), carbomer, polyacrylic acid (PAA), PLGA (poly(lactide-co-glycolide)), PHEMA (poly(2-hydroxyethyl methacrylate)), polylactic acid (PLA), polyurethane, PDMAAm (poly(N,N-dimethylacrylamide)), PGS (poly(glycerol sebacate)), poly(glycolyglucan), poly(acryloyl-glucosamine), PAE (poly(acryloyloxyethyltrimethyl ammonium chloride)), furan derivatives, and poly(N,N-bis(methacryloyl)amino acid).

4. The method of claim 3, wherein the solute of the second aqueous solution is at least one selected from the group consisting of:
polyethylene glycol (PEG), propylene glycol (PPG), polyvinylpyrrolidone (PVP), polyvinyl alcohol (PVOH, PVA, or PVAI), poly(vinyl methyl ether) (PVME), hydroxypropyl starch (HP-starch), UCON, polyethersulfone (PES), pullulan, polypropylene glycol dimethyl ether (PPGDME), dextran (DEX), maltodextrin, hydroxyethylcellulose (Natrosol), polyethylene glycol methyl ether (PEGME), polyethylene glycol dimethyl ether (PEGDME), polyacrylamide (PAM), poly(styrene sulfonate) (PSS), DEAE-dextran, polyethyleneimine (PEI), hydroxypropyl methylcellulose (HPMC), poly(N-isopropylacrylamide) (PNIPAM), poly(diallyldimethylammonium chloride) (PDADMAC), polyvinyl caprolactam (PVCL), and Ficoll.

5. The method of claim 4, wherein a weight percentage of the solute of the first aqueous solution is from 0.1% to 5% (w/w).

6. The method of claim 5, wherein an average molecular weight of the solute of the first aqueous solution is from 1,000 Da to 500,000 Da.

7. The method of claim 6, wherein an average molecular weight of the solute of the first aqueous solution is from 10 kDa to 50,000 kDa.

8. The method of claim 7, wherein a concentration of the solute of the first aqueous solution is at least 0.16% by weight.

9. The method of claim 4, wherein a weight percentage of the polyethylene glycol (PEG) is from 3% to 50% (w/w).

10. The method of claim 9, wherein a ratio of the average molecular weight (kDa) of the solute of the first aqueous solution to the weight percentage (w/w) of the solute of the second aqueous solution is from 5 kDa/(w/w) to 1,000 kDa/(w/w).

11. The method of claim 4, wherein the cationic salt comprises a cation selected from the group consisting of monovalent cations, divalent cations, and trivalent cations.

12. The method of claim 11, wherein a weight percentage of the cationic salt is from 0.5% to 20% (w/w).

13. The method of claim 11, wherein the monovalent cation is selected from the group consisting of *Na*⁺, *K*⁺, and *Ag*⁺.

14. The method of claim 11, wherein the divalent cation is selected from the group consisting of *Ca*²⁺, *Mg*²⁺, *Ba*²⁺, and *Pb*²⁺.

15. The method of claim 11, wherein the trivalent cation is an *Al*³⁺ cation.

16. The method of claim 11, wherein the biological material is at least one selected from the group consisting of mesenchymal stem cells (MSCs), MSC-derived exosomes, viruses, proteins, RNA, platelet-rich plasma (PRP), and regeneration factors.

17. The method of claim 16, wherein the regeneration factor is at least one selected from the group consisting of:
bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), plateletderived growth factor (PDGF), placental growth factor (PlGF), and hepatocyte growth factor (HGF).

18. The method of claim 16, wherein the protein is a protein-derived anticancer agent.

19. The method of claim 16, wherein the biological material is a genomicbased anticancer agent.

20. The method of claim 16, wherein the biological material is a vaccine.

21. The method of claim 20, wherein the vaccine is for a disease selected from the group consisting of:
anthrax, diphtheria, meningitis, hepatitis A, hepatitis B, influenza, Japanese encephalitis, Lyme disease, measles, mumps, pertussis, pneumococcal disease, polio, rabies, hemorrhagic fever with renal syndrome, rotavirus, rubella, shingles, smallpox, tetanus, tuberculosis, typhoid fever, varicella, Ebola hemorrhagic fever, yellow fever, cholera, human papillomavirus (HPV), and COVID-19.

22. The method of claim 4, wherein the first aqueous solution serves as a structural framework for forming the hydrogel.

23. A hydrogel prepared by the method of any one of claims 1 to 22.

24. A method for skin aesthetic treatment, comprising administering to a subject a hydrogel prepared by the method of any one of claims 1 to 22.

25. A composition for skin aesthetics, comprising a hydrogel prepared by the method of any one of claims 1 to 22.

26. A cosmetic comprising the skin aesthetic composition of claim 25.

27. A composition for wound healing, comprising a hydrogel prepared by the method of any one of claims 1 to 22.

28. The composition of claim 27, wherein the wound is selected from the group consisting of:
traumatic wounds, pressure sores (decubitus ulcers), burns, abrasions, puncture wounds, ulcerative wounds, stab wounds, chronic skin wounds caused by reactive oxygen species (ROS), contusions (bruises), incised wounds (cuts), wounds of the throat or oral mucosa, lacerations, diabetic ulcers, leg ulcers, hypertensive ischemic ulcers, venous ulcers, and foot ulcers.

29. A composition for regeneration, comprising a hydrogel prepared by the method of any one of claims 1 to 22.

30. The composition of claim 29, wherein the regeneration is for a condition selected from the group consisting of:
temporomandibular disorders, gastric ulcers caused by endoscopic submucosal dissection (ESD), human autologous fat grafting, knee osteoarthritis, chronic non-healing ulcers, hair regeneration therapy for androgenetic alopecia, Achilles tendon rupture, high-risk proliferative diabetic retinopathy, patellar dislocation, lumbar facet joint syndrome, retinitis pigmentosa, diabetic macular edema, traumatic bone cysts, scars, dry eye syndrome, Sjögren's syndrome, osteoarthritis, meniscal lesions, melasma, muscle injury, ulnar neuropathy at the elbow, chronic ulcers, alopecia, epicondylitis, bone resorption, rotator cuff tears, thin endometrium, striae gravidarum, plantar fasciitis, coxarthrosis, muscle rupture, tennis elbow, skin grafting, aging, knee arthritis, burns, renal failure, and atopic diseases.

31. A container for extracting or concentrating particles, the container being used for preparing a hydrogel according to any one of claims 1 to 22, the container comprising:
a body providing an internal space configured to accommodate an aqueous solution for aqueous two-phase system and to extract or concentrate the particles therein during centrifugation;
a first lid provided at one side of the body for injecting blood or a separate substance into the internal space of the body; and
a second lid provided at the other side of the body opposite to the one side for discharging the particles extracted or concentrated in the body.

32. The container of claim 31, wherein the particles are at least one selected from the group consisting of mesenchymal stem cells (MSCs), MSC-derived exosomes, viruses, proteins, RNA, and regeneration factors.

33. The container of claim 32, wherein the regeneration factor is at least one selected from the group consisting of:
bone morphogenetic protein (BMP), fibroblast growth factor (FGF), vascular endothelial growth factor (VEGF), nerve growth factor (NGF), epidermal growth factor (EGF), insulin-like growth factor (IGF), transforming growth factor (TGF), brain-derived neurotrophic factor (BDNF), plateletderived growth factor (PDGF), placental growth factor (PlGF), and hepatocyte growth factor (HGF).

34. The container of claim 31, wherein the first lid is provided on a cover disposed at an upper side of the body to open and close the body.

35. The container of claim 34, wherein the other side of the body is tapered toward the second lid.

36. The container of claim 35, further comprising a support surrounding an outer surface of the other side of the body around the second lid.
